(19) Europäisches Patentamt / European Patent Office / Office européen des brevets

(11) **EP 2 207 416 B1**

(12) **EUROPEAN PATENT SPECIFICATION**

(45) Date of publication and mention
of the grant of the patent:
**07.11.2018 Bulletin 2018/45**

(21) Application number: **08835991.4**

(22) Date of filing: **29.09.2008**

(51) Int Cl.:
*A01N 25/08* (2006.01)     *A01N 25/10* (2006.01)
*A61K 8/25* (2006.01)      *A01N 65/00* (2009.01)
*A01N 65/26* (2009.01)     *A01N 25/26* (2006.01)
*A61K 8/58* (2006.01)      *A61Q 17/00* (2006.01)
*A61K 8/92* (2006.01)      *A61K 8/96* (2006.01)
*A61K 8/02* (2006.01)      *C09C 3/12* (2006.01)
*C09C 1/02* (2006.01)      *C09C 1/42* (2006.01)

(86) International application number:
**PCT/US2008/078075**

(87) International publication number:
**WO 2009/045941 (09.04.2009 Gazette 2009/15)**

(54) **ENHANCED RETENTION CAPABILITIES THROUGH METHODS COMPRISING SURFACE TREATMENT OF FUNCTIONAL PARTICULATE CARRIER MATERIALS, AND FUNCTIONAL PARTICULATE CARRIER MATERIALS MADE THEREFROM**

ERHÖHTE RÜCKHALTEFÄHIGKEIT DURCH VERFAHREN MIT OBERFLÄCHENBEHANDLUNG FUNKTIONELLER PARTIKELTRÄGERMATERIALIEN UND DARAUS HERGESTELLTE FUNKTIONELLE PARTIKELTRÄGERMATERIALIEN

MEILLEURES CAPACITÉS DE RÉTENTION GRÂCE À DES PROCÉDÉS COMPRENANT UN TRAITEMENT DE SURFACE DE MATÉRIAUX SUPPORTS PARTICULAIRES FONCTIONNELS, ET MATÉRIAUX SUPPORTS PARTICULAIRES FONCTIONNELS RÉALISÉS À PARTIR DE CEUX-CI

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **02.10.2007 US 976950 P**

(43) Date of publication of application:
**21.07.2010 Bulletin 2010/29**

(73) Proprietor: **Imerys Filtration Minerals, Inc.
San Jose, CA 95112 (US)**

(72) Inventors:
• **GREENE, Michael
Goleta
CA 93117 (US)**

• **LU, Jie
Lompoc
CA 93436 (US)**

(74) Representative: **Rushton, David John et al
Haseltine Lake LLP
Redcliff Quay
120 Redcliff Street
Bristol BS1 6HU (GB)**

(56) References cited:
EP-A1- 0 364 601      WO-A2-2007/015243
GB-A- 2 026 866       US-A- 3 207 699
US-A- 4 855 054       US-A- 4 889 747
US-A1- 2005 211 635   US-A1- 2007 048 344
US-A1- 2007 065 475   US-A1- 2007 065 475
US-B1- 6 905 698      US-B1- 6 905 698
US-B2- 7 018 643      US-B2- 7 018 643

**Description**

**FIELD OF THE INVENTION**

This application and the inventions described herein discuss functional particulate carrier materials with enhanced retention capabilities.

**BACKGROUND OF THE INVENTION**

Preventing microbial contamination is an essential concern in daily life, in everywhere from surface areas in bathrooms, to surgical instruments, to wall paints. U.S. Patent No. 6,905,698 B1 generally discusses certain methods by which biocides can be used. Furthermore, incorporating active ingredients, such as biocides, into plastics and liquid-based media by way of particulate carrier materials can prevent microbial contamination in a variety of applications, and the functional particulate carrier materials can ideally incorporate the active ingredients into the finished products by additionally fulfilling, and without compromising, other filler functions. However, the effectiveness of the antimicrobial capabilities may decrease over time as the biocides diffuse out of the embedded particulate carrier materials.

Introducing active ingredients such as biocides into or with carrier materials has apparently been disclosed for at least some variety of applications, such as in U.S. Patent Nos. 4,505,889, 4,552,591, 5,648,086, and 7,018,643, in U.S. Patent Application Publication Nos. 2006/0035097 and 2006/0180552, and in the use of celite as a biocide carrier. Historically, however, functional particulate carrier materials have failed to retain large amounts of the active ingredients (e.g., biocides), limiting the antimicrobial capabilities of the products, both in their initial antimicrobial capability and their continued antimicrobial capability as the active ingredient may diffuse from the carrier materials. Current practices attempt to prolong the antimicrobial capabilities of different materials by slowing the diffusivity of the active ingredients in various ways. U.S. Patent Application No. 2006/0246149 A1 discusses a protective surface coating; U.S. Patent No. 6,905,698 B1 also discusses a protective surface coating or surface cleaning compositions; U.S. Patent No, 4,656,057 attempts to solve the problem through use of porous or perforated membranes. The impregnation of mineral particles with biocides is also known; for example, U.S. Patent No. 4,552,591 describes a composition intended to protect polymer dispersions used in oil field water treatment. However, none of those references appear to contemplate a surface treatment that modifies the surface of the functional particulate carrier materials to increase the retention of active ingredients as a method to enhance the antimicrobial capabilities of the functional particulate carriers.

**SUMMARY**

The present invention concerns a surface treated functional particulate carrier material, comprising: at least one functional particulate carrier material subjected to at least one surface treatment; and,at least one active ingredient,wherein the at least one functional particulate carrier material is chosen from the group consisting of synthetic calcium silicate hydrate, salts thereof, and diatomaceous earth and wherein the at least one surface treatment is a chemical surface modification with at least one surface treating agent chosen from the group consisting of at least one silane wherein the at least one silane is trialkoxysilane, and wherein the at least one surface treated functional particulate carrier material absorbs the at least one active ingredient or the at least one active ingredient adsorbs onto the surface of the at least one surface treated functional particulate carrier material. Disclosed herein are functional particulate carrier materials with enhanced retention capabilities and methods for enhancing the performance of plastics and liquid-based media, for example, in coatings and paints, by increasing the retention factor of at least one active ingredient through at least one surface treatment of at least one functional particulate carrier material. Methods for using the functional particulate carrier materials with enhanced antimicrobial capabilities are also disclosed.

**DETAILED DESCRIPTION**

At least one functional particulate carrier material is surface treated to chemically modify its surface prior to exposure to at least one active ingredient with antimicrobial capability. The at least one surface treatment may allow for higher retention of the at least one active ingredient, thereby enhancing the antimicrobial capability of the at least one functional particulate carrier material.

Functional Particulate Carrier Material

At least one functional particulate carrier material is used as the material subjected to at least one surface treatment, prior to exposure to an at least one active ingredient. Combinations of functional particulate carrier materials may be used. The skilled artisan will readily understand appropriate functional particulate carrier materials appropriate for use in the disclosure described herein. In one embodiment, the functional particulate carrier material is any inorganic substrate whose surface is capable of being modified through an at least one surface treatment to allow chemical bonding with at least one active ingredient.

In an embodiment of the invention, the functional particulate carrier material is diatomaceous earth. Diatomaceous earth is a sedimentary silica deposit comprising the fossilized skeletons of diatoms, which are one-celled algae-like plants that accumulate in marine or fresh water environments. Honeycomb silica structures generally give diatomaceous earth useful characteristics such as absorptive capacity and surface area, chemical stability, and low bulk density. In one embodiment, diatomaceous earth comprises about 90% $SiO_2$ mixed with other substances. In another embodiment, diatomaceous earth comprises about 90% $SiO_2$, plus various metal oxides, such as but not limited to Al, Fe, Ca, and Mg oxides. In one embodiment, the diatomaceous earth is natural, i.e., unprocessed. In another embodiment, the diatomaceous earth is calcined. In yet another embodiment, the diatomaceous earth is flux calcined. In a further embodiment, diatomaceous earth is a commercially available super-fine diatomaceous earth product, such as but not limited to Superfloss™ available from Celite Corporation. In yet another embodiment, the diatomaceous earth is CelTiX™, available from World Minerals Inc.

In a further embodiment of the disclosure the functional particulate carrier material is perlite. Perlite, as used herein, identifies any naturally occurring siliceous volcanic rock that can be expanded with heat treatment. In one embodiment, perlite comprises between about 70% and about 74% silica, about 14% alumina, between about 2% and 6% water, and trace impurities. In one embodiment of the disclosure, the perlite is ore. In another embodiment of the disclosure, the perlite is expanded. In yet another embodiment, the perlite is fine. In still another embodiment, the perlite is Harborlite 635, a very fine grade of perlite available from Harborlite Corp., a subsidiary of World Minerals Inc.

In yet another embodiment of the disclosure, the functional particulate carrier material is kaolin clay, which may also be referred to as china clay or hydrous kaolin. In one embodiment, kaolin clay comprises predominantly mineral kaolinite ($Al_2Si_2O_5(OH)_4$), anhydrous aluminum silicate, and amounts of various impurities. Exemplary kaolin clays include, but are not limited to, airfloat kaolin clay, water-washed kaolin clay, delaminated kaolin clay, and calcined kaolin clay.

In yet a further embodiment of the disclosure, the functional particulate carrier material is glass. In still another embodiment, the carrier material is a mineral filler for plastics. In still a further embodiment, the material is a metallic oxide. In an embodiment of the invention, the functional particulate carrier material is a synthetic calcium silicate hydrate ($CaSiO_3$). An exemplary synthetic $CaSiO_3$ is Micro-Cel E, available from Advanced Minerals Corp., a subsidiary of World Minerals Inc. In yet another embodiment of the disclosure, the functional particulate carrier material is vermiculite. In yet a further embodiment of the disclosure, the functional particulate carrier material is a phyllosilicate.

In one embodiment of the disclosure, the functional particulate carrier material is talc. In another embodiment of the disclosure, the functional particulate carrier material is talc comprising greater than about 90% $Mg_3Si_4O_{10}(OH)_2$ (magnesium silicate hydroxide) and accessory minerals in varying amounts, including, but not limited to, chlorite, serpentine, quartz, tremolite, anthophyllite, and carbonates such as magnesite, dolomite, and calcite. In a further embodiment of the disclosure, the functional particulate carrier material is platy talc. In yet another embodiment of the disclosure, the functional particulate carrier material is industrial talc. In yet a further embodiment of the disclosure, the functional particulate carrier material is tremolitic talc.

In one embodiment of the disclosure, the functional particulate carrier material is mica. In another embodiment, the functional particulate carrier material is mica with the general formula $X_2Y_{4-6}Z_8O_{20}(OH,F)_4$, in which: X may be, but is not limited to, K, Na, Ca, Ba, Rb, or Cs; Y may be, but is not limited to, Al, Mg, Fe, Mn, Cr, Ti, and Li; and, Z may be, but is not limited to, Si, Al, Fe, and Ti.

In another embodiment of the disclosure, the functional particulate carrier material is selected from the group consisting of, but not limited to, activated carbon, powders of polyethylene, fibers of polyethylene, fibers of polypropylene, high aspect ratio Wollastonite, low aspect ratio Wollastonite, amorphous silicas, amorphous aluminas, alumina trihydrate, barite (Barium Sulfate), ground calcium carbonate, precipitated calcium carbonate, calcium sulfate, gypsum, carbon black, clay, chlorite, dolomite, feldspar, graphite, huntite, hydromagnesite, hydrotacite, magnesia, magnesite (magnesium carbonate), magnesium hydroxide, magnetite ($Fe_3O_4$), nepheline syenite, olivine, pseudoboehmites (forms of microcrystalline aluminum hydroxide), pyrophyllite, resins, titania, titanium dioxide (e.g., rutile), waxes, zeolites (e.g., Y-zeolites and dealuminated Y-zeolites), and zinc oxide.

In another embodiment of the disclosure, the functional particulate carrier material is silica. Examples of silica include, but are not limited to, ground silica, novoculite silica, precipitated silica, fumed silica, and fumed amorphous silica. In a further embodiment of the disclosure, the functional particulate carrier material is synthetic silica. Examples of synthetic silicas include, but are not limited to, silica gels, silica colloids, synthetic fused silica, and doped synthetic fused silica. In yet another embodiment of the disclosure, the functional particulate carrier material is an aluminosilicate, with the basic structural composition $AlSiO_4$. Exemplary aluminosilicates include, but are not limited to, calcium aluminosilicate, sodium aluminosilicate, potassium aluminosilicate, zeolite, and kyanite.

Surface Treatment

At least one surface treatment is used to modify the surface of the at least one functional particulate carrier material. In one embodiment of the disclosure, the at least one surface treatment at least partially chemically modifies the surface of the at least one functional particulate carrier material by way of at least one surface treating agent. Chemical modification includes, but is not limited to, covalent bonding, ionic bonding, and "weak" intermolecular bonding such as van der Waals interactions. In another embodiment, the at least one surface treatment at least partially physically modifies the surface of the at least one functional particulate carrier material. Physical modification includes, but is not limited to, roughening of the material surface, pitting the material surface, or increasing the surface area of the material surface. In a further embodiment, the at least one surface treatment at least partially chemically modifies and at least partially physically modifies the surface of the at least one functional particulate carrier material. In yet another embodiment, the at least one surface treatment is any chemical or physical modification to the surface of the at least one functional particulate carrier material that results in increased retention of at least one active ingredient.

In one embodiment of the disclosure, the at least one surface treatment silanizes the at least one functional particulate carrier material, wherein the at least one surface treating agent is at least one siloxane. In general, siloxanes are any of a class of organic or inorganic chemical compounds comprising silicon, oxygen, and often carbon and hydrogen, based on the general empirical formula of $R_2SiO$, where R may be an alkyl group. Exemplary siloxanes include, but are not limited to, dimethylsiloxane, methylphenylsiloxane, methylhydrogen siloxane, methyltrimethoxysilane, octamethylcyclotetrasiloxane, hexamethyldisiloxane, diphenylsiloxane, and copolymers or blends of copolymers of any combination of monophenylsiloxane units, diphenylsiloxane units, phenylmethylsiloxane units, dimethylsiloxane units, monomethylsiloxane units, vinylsiloxane units, phenylvinylsiloxane units, methylvinylsiloxane units, ethylsiloxane units, phenylethylsiloxane units, ethylmethylsiloxane units, ethylvinylsiloxane units, or diethylsiloxane units.

In one embodiment of the disclosure, the at least one surface treatment silanizes the at least one functional particulate carrier material, wherein the at least one surface treating agent is at least one silane. In general, silanes and other monomeric silicon compounds have the ability to bond inorganic materials, such as at least one functional particulate carrier material, to organic resins and materials, such as at least one active ingredient. The bonding mechanism may be due largely to two groups in the silane structure: the $Si(OR_3)$ portion interacts with the at least one inorganic functional particulate carrier material, while the organofunctional (vinyl-, amino-, epoxy-, etc.) group interact with the at least one active ingredient.

In one embodiment of the disclosure, at least one functional particulate carrier material is subjected to at least one surface treatment surface treated with at least one ionic silane. Exemplary ionic silanes include, but are not limited to, 3-(trimethoxysilyl)propyl-ethylenediamine triacetic acid trisodium salt and 3-(trihydroxysilyl)propylmethylposphonate salt. In another embodiment, the carrier material is subjected to at least one surface treatment with at least one nonionic silane. In a further embodiment, the carrier material is subjected to at least one surface treatment with at least one silane of Formula (I):

$$(R^1)_x Si(R^2)_{3-x} R^3 \qquad (I)$$

wherein: $R^1$ is any hydrolysable moiety that may chemically react with any active group on the surface of the at least one functional particulate carrier material, such as but not limited to alkoxy, halogen, hydroxy, aryloxy, amino, amide, methacrylate, mercapto, carbonyl, urethane, pyrrole, carboxy, cyano, aminoacyl, or acylamino, alkyl ester, and aryl ester; X has a value between 1 and 3, such that more than one siloxane bond may be formed between the at least one functional particulate carrier material and the at least one silane; $R^2$ is any carbon-bearing moiety that does not substantially react or interact with the at least one functional particulate carrier material during the treatment process, such as but not limited to substituted or unsubstituted alkyl, alkenyl, alkaryl, alkcycloalkyl, aryl, cycloalkyl, cycloalkenyl, heteroaryl, heterocyclic, cycloalkaryl, cycloalkenylaryl, alkcycloalkaryl, alkcycloalkenyaryl, and arylalkaryl; $R^3$ is any organic containing moiety that remains substantially chemically attached to the silicon atom of Formula (I) once the at least one surface treatment is completed and that is capable or reacting or interacting with the at least one active ingredient, such as but not limited to hydrogen, alkyl, alkenyl, alkaryl, alkcycloalkyl, aryl, cycloalkyl, cycloalkenyl, heteroaryl, heterocyclic, cycloalkaryl, cycloalkenylaryl, alkcycloalkaryl, alkcycloalkenyaryl, arylalkaryl, alkoxy, halogen, hydroxy, aryloxy, amino, amide, methacrylate, mercapto, carbonyl, urethane, pyrrole, alkyl ester, aryl ester, carboxy, sulphonate, cyano, aminoacyl, acylamino, epoxy, phosphonate, isothiouronium, thiouronium, alkylamino, quaternary ammonium, trialkylammonium, alkyl epoxy, alkyl urea, alkyl imidazole, or alkylisothiouronium; wherein the hydrogen of said alkyl, alkenyl, aryl, cycloalky, cycloalkenyl, heteroaryl, and heterocyclic is optionally substituted by, for example, halogen, hydroxy, amino, carboxy, or cyano.

In another embodiment of the disclosure, at least one functional particulate carrier material with a hydroxyl-bearing porous surface is subjected to at least one surface treatment with at least one silane, such that the material surface is covalently bonded to the at least one silane. In such an embodiment, the surface area of the at least one functional particulate carrier material may limit the amount of the bound silane and, as a result, it may be preferable to subject the carrier material to at least one physical surface treatment that increases the surface area of the carrier material prior to treatment with the at least one silane.

In a further embodiment of the disclosure, the at least one functional particulate carrier material is subjected to at least one surface treatment with at least one silane having one or more moieties selected from the group consisting of alkoxy, quaternary ammonium, aryl, epoxy, amino, urea, methacrylate, imidazole, carboxy, carbonyl, isocyano, isothiorium, ether, phosphonate, sulfonate, urethane, ureido, sulfhydryl, carboxylate, amide, pyrrole, and ionic.

Exemplary silanes having an alkoxy moiety include, but are not limited to, are mono-, di-, or trialkoxysilanes, such as n-octadecyltriethoxysilane, n-octytriethoxysilane, and phenyltriethoxysilane.

Exemplary silanes having a quaternary ammonium moiety include, but are not limited to, quaternary ammonium salts of a substituted silanes, 3-(trimethoxysilyl) propyloctadecyldimethylammonium chloride, poly-(diallyldimethylammonium chloride), N-trimethoxysilylpropyl-N,N,N-trimethylammonium chloride, octadecylaminodimethyl trimethoxysilylpropyl ammonium chloride, and 3-(N-styrylmethyl-2-aminoethylamino)-propyltrimethoxysilane hydrochloride.

Exemplary silanes having an aryl moiety include, but are not limited to, 3- (trimethoxysilyl)-2-(p,m-chloromethyl)-phenylethane, 2-hydroxy-4-(3-triethoxysilylpropoxy)-diphenylketone, ((chloromethyl)phenylethyl)trimethoxysilane and phenyldimethylethoxysilane.

Exemplary silanes having an epoxy moiety include, but are not limited to, 3-glycidoxypropyltrimethoxysilane and 2-(3,4-epoxycyclohexyl) ethyltrimethoxysilane.

Exemplary silanes having an amino moiety include, but are not limited to, 3-aminopropyltrimethoxysilane, N-(2-aminoethyl)-3-aminopropyltrimethoxysilane, trimethoxysilylpropyldiethylenetriamine, 2-(trimethoxysilylethyl) pyridine , N-(3-trimethoxysilylpropyl)pyrrole, trimethoxysilylpropyl polyethyleneimine, bis-(2-hydroxyethyl)-3-aminopropyltriethoxysilane, and bis(2-hydroxyethyl)-3-amino propyltriethoxysilane.

Exemplary silanes having a urea moiety include, but are not limited to, N- (triethoxysilylpropyl)urea and N-1-phenylethyl-N'-triethoxysilylpropylurea.

A nonlimiting example of a silane having a methacrylate moiety is 3-(trimethoxysilyl)propyl methacrylate.

Exemplary silanes having an imidazole moiety include, but are not limited to, N-[3-(triethoxysilyl)propyl]imidazole and N-(3-triethoxysilylpropyl)-4,5-dihydroimidazole.

A nonlimiting example of a silane having a carbonyl moiety is 3-(triethoxysilyl)propylsuccinate.

Exemplary silanes with an isocyano moiety include, but are not limited to, tris(3-trimethoxysilylpropyl)isocyanurate and 3-isocyanatopropyltriethoxysilane.

A nonlimiting example of a silane having an isothiourium moiety is the salt of trimethoxysilylpropylisothiouronium, such as the chloride salt.

Exemplary silanes having an ether moiety include, but are not limited to, bis[(3-methyldimethoxysilyl)propyl]-polypropylene oxide and N-(triethoxysilylpropyl)-O- polyethylene oxide urethane.

A nonlimiting example of a silane having a sulfonate moiety is 2-(4-chlorosulfonylphenyl)-ethyltrichlorosilane.

Exemplary silanes having a urethane moiety include, but are not limited to, N-(triethoxysilylpropyl)-O-polyethylene oxide urethane and O-(propargyloxy)-N-(triethoxysilylpropyl) urethane.

A nonlimiting example of a silane having a sulfhydryl moiety is 3-mercaptopropyltriethoxysilane.

Exemplary silanes having an amide moiety include, but are not limited to, triethoxysilylpropylethyl-carbamate, N- (3-triethoxysilylpropyl)-gluconamide, N-(triethoxysilylpropyl) -4-hydroxybutyramide.

In yet another embodiment of the disclosure, the at least one functional particulate carrier material is subjected to surface treatment with a combination of silanes, such as but not limited to: N- trimethoxysilylpropyl-N, N,N-trimethylammonium chloride and bis(2-hydroxyethyl)-3- aminopropyltriethoxysilane; 3-aminopropyltrimethoxysilane and N-(triethoxysilylpropyl)-O-polyethylene oxide urethane; 3-trihydrosilylpropyhnethylphosphonate, sodium salt, and N-(triethoxysilylpropyl) -O-polyethylene oxide urethane; N-trimethoxysilylpropyl-N,N,N-Cl, trimethylammonium chloride and (3-glycidoxypropyl) trimethoxysilane; 3-trihydrosilylpropylmethylphosphonate, sodium salt and bis-(2-hydroxyethyl)-3-aminopropyltriethoxysilane; 3-(N-styrylmethyl-2-aminoethylamino)-propyltrimethoxysilane hydrochloride and N- (triethoxysilylpropyl)-O-polyethylene urethane; 2- (trimethoxysilylethyl) pyridine and N- (3-triethoxysilylpropyl)-gluconamide; trimethoxysilylpropyl-N, N,N-Cl, trimethylammonium chloride and N-(3-triethoxysilylpropyl) -gluconamide; N-trimethoxysilylpropyl-N, N,N-Cl, trimethylammonium chloride and 2-hydroxy-4- (3-triethoxysilylpropoxy)-diphenylketone; mercaptopropyltriethoxysilane and N-(triethoxysilylpropyl)-O-polyethylene oxide urethane; 3- (triethoxysilyl)propylsuccinic and N-(triethoxysilylpropyl)-O-polyethylene urethane, trimethoxysilylpropyl-ethylenediamine, triacetic acid, trisodium salt and N-(triethoxysilylpropyl) -O-polyethylene oxide urethane; 2-(4-chlorosulfonylphenyl)-ethyltrichlorosilane and N-(triethoxysilylpropyl)-O-polyethylene oxide urethane; and 2-(4- chlorosulfonylphenyl) -ethyltrichlorosilane and bis-(2-hydroxyethyl)-3-aminopropyltriethoxysilane.

In some embodiments, silanization may proceed according to "wet" or "dry" processes well-known to the skilled artisan. For example, a "wet" process generally comprises reacting the at least one silane onto the at least one functional particulate carrier material in at least one solvent (e.g., organic solvent or water). In one embodiment, heat is used in place of or in addition to the at least one solvent. Heat or solvent is not required for the "wet" process, but it may improve the reaction rate and the uniform surface coverage. In another embodiment, a "wet" process includes in-line mixing of slurries or liquids during typical silanization processing steps, including but not limited to filtration and drying.

In one embodiment, a "dry" process generally comprises reacting the at least one silane with the at least one functional particulate carrier material in a vapor phase by mixing the at least one silane with the at least one functional particulate carrier material and then heating the mixture. In another embodiment, a "dry" process comprises reacting the at least one silane with the at least one functional particulate carrier material in a stirred liquid phase by mixing the at least one silane with the at least one functional particulate carrier material and then heating the mixture. In one embodiment, a "dry" process comprises mixing at least one silane with at least one functional particulate carrier material and incubating in a sealed container at elevated temperatures to speed up the surface treatment process. In yet another embodiment, the "dry" process includes mixing of dry powdered functional particulate carrier materials and a liquid silane additive, where the amount of silane added is small enough that the reaction mass remains solid-like and can continue to be processed like a dry powder.

In one embodiment, the at least one functional particulate carrier material is subjected to at least one surface treatment with at least one silane by adding the at least one silane gradually to a rapidly stirred solvent, which is in direct contact with the at least one functional particulate carrier material. In another embodiment, the at least one functional particulate carrier material is subject to at least one surface treatment with at least one silane by carrying out the treatment in a vapor phase, which causes the vapor of the at least one silane to contact and react with the at least one functional particulate carrier material.

In another embodiment, the at least one functional particulate carrier material is placed in a vacuum reactor and dried under vacuum. At least one silane may then be added to the vacuum chamber as a vapor and contact the at least one functional particulate carrier material. After a certain contact time, byproducts of the reaction may be removed under reduced pressure. When the vacuum is released, the surface treated carrier material may be removed from the chamber. The actual treatment process may be carried out in a period from about 1 minute to about 24 hours. The treatments can be carried out at temperatures ranging from about 0 °C to about 400 °C.

The amount of at least one silane used in the at least one surface treatment may depend on various factors, including but not limited to the amount of the at least one carrier material to be surface treated, the number of hydroxyl groups on the surface of the at least one carrier material to be reacted, and the molecular weight of the at least one silane. In one embodiment, a stoichiometric amount equivalent to the available surface hydroxyls plus some excess amount of the at least one silane is used for the at least one surface treatment, in an effort to reduce the number of potential side reactions. In another embodiment, greater than a stoichiometric amount of at least one silane is used to create a thicker or more dense surface treatment. In one embodiment, about 0 to about 500 times stoichiometric excess is used. In another embodiment, about 5 to about 100 times stoichiometric excess is used. In a further embodiment, about 10 to about 50 times stoichiometric excess is used. In yet another embodiment, about 10 to about 20 times stoichiometric excess is used.

In some embodiments, at least one silane with at least one hydrolysable group may condense with at least one hydroxyl group on the surface of the at least one functional particulate carrier material and provide covalent coupling of organic groups to those substrates. In one embodiment, at least one alkoxy group of the at least one silane chemically reacts with at least one hydroxyl group on the surface of the at least one functional particulate carrier material. In another embodiment, at least one silane having at least one quaternary ammonium moiety is used and the protonated, positive charge of those silanes electrostatically attract to at least one deprotonated group of the at least one functional particulate carrier material to facilitate fast and efficient reaction.

Surface Treated Functional Particulate Carrier Materials

Further disclosed herein is at least one surface treated functional particulate carrier material, which is produced from at least one surface treatment of at least one functional particulate carrier material. In one embodiment, the silanized functional particulate carrier materials have the general formula selected from the group consisting of Formulas (II) to (IV):

$$\text{Particle-O-Si}(R^1)_x(R^2)_{3-x}R^3 \qquad \text{(II)}$$

$$\text{Particle-O-Si}(R^1)_x(R^2)_{3-x}R^4NR^5\text{-CH}_2\text{-C}(\text{-OR}^5)R^6\text{-R}^7N^+(R^8)_3Cl^- \qquad \text{(III)}$$

$$\text{Particle-O-Si}(R^1)_x(R^2)_{3-x}R^4OR^9C(\text{-OH})R^6\text{--CH}_2SO_3^-Na^+ \qquad \text{(IV)}$$

wherein $R^1$, $R^2$, $R^3$, and x may be the same as described above for Formula (I), so long as no more than four groups directly attach to the silicon atom; $R^5$, $R^6$, $R^8$ may be independently hydrogen, substituted or unsubstituted alkyl, alkenyl, alkaryl, alkcycloalkyl, aryl, cycloalkyl, cycloalkenyl, heteroaryl, heterocyclic, cycloalkaryl, cycloalkenylaryl, alkcycloalkaryl, alkcycloalkenyaryl, ether, ester or arylalkaryl; and, $R^4$, $R^7$, $R^9$ may be substituted or unsubstituted alkyl, alkenyl, alkaryl, alkcycloalkyl, aryl, cycloalkyl, cycloalkenyl, heteroaryl, heterocyclic, cycloalkaryl, cycloalkenylaryl, alkcycloalkaryl, alkcycloalkenyaryl, or arylalkaryl radicals capable of forming two covalent attachments.

In some embodiments of the disclosure, the product of at least one functional particulate carrier material subjected to at least one surface treatment with at least one silane may have at least one functional moiety. Exemplary functional moieties include, but are not limited to, a selection from the group consisting of quaternary ammonium, epoxy, amino, urea, methacrylate, imidazole, sulphonate, and other organic moieties known to react with biological molecules. In one embodiment, the functionally moiety is further reacted, using well-known methods to create further new functionalities for other interactions.

Surface treated functional particulate carrier materials made according to the present disclosure may be characterized, for instance, by measuring surface area, pore volume, and pore size using methods known to the art, such as a Micrometrics analyzer. For example, surface area can be characterized by BET technique. Pore volume and pore diameter can be calculated by Barrett-Joyner-Halenda analysis. Specific functional groups and molecular structure can be determined by NMR and IR spectroscopy. Carbon-hydrogen-nitrogen content can be determined by combustion techniques; from that analysis information, the treatment level on the particle surface can be calculated. The skilled artisan readily understands that the embodiments disclosed herein may utilize any method of characterizing the functional particulate carrier materials.

Uses for Surface Treated Functional Particulate Carrier Materials

The surface treated functional particulate carrier materials produced according to the present disclosure may be used in a variety of applications. In one embodiment, methods to use the surface treated functional particulate carrier materials to bind at least one active ingredient are disclosed. In another embodiment, surface treated functional particulate carrier materials are used to enhance the antimicrobial activity by capturing soluble active ingredients through electrostatic, hydrophobic, and/or hydrophilic interaction mechanisms.

In another embodiment of the disclosure, specific charged groups have been attached covalently to the surface of the functional particulate carrier materials so they can be used to capture at least one active ingredient electrostatically. The oppositely charged materials may, therefore, be bound to the porous treated surface. In a further embodiment, in addition to the electrostatic attraction, hydrophobic or hydrophilic ligands are used to improve the binding and/or release characteristics of the functional particulate carrier materials by hydrophobic or hydrophilic interaction. The skilled artisan readily understands that the embodiments disclosed herein may utilize any method to use the surface treated functional particulate carrier materials to bind to active ingredients.

Active Ingredient

At least one active ingredient may bind to or otherwise interact with the surface treated functional particulate carrier materials made according to the present disclosure. In one embodiment, the surface treated functional particulate carrier material absorbs the at least one active ingredient. In another embodiment, the surface treated functional particulate carrier material bonds to the at least one active ingredient.

The at least one active ingredient may take any of various forms and fulfill any of various functions. In one embodiment, the at least one active ingredient is any substance that will bind to or otherwise interact with at least one functional particulate carrier material that has been subjected to at least one surface treatment. In another embodiment, the at least one active ingredient is any substance that will bind to or otherwise interact with at least one functional particulate carrier material that has been subjected to at least one surface treatment, the activated product of which is useful as an additive to substances such paints, resins, and plastics. In a further embodiment, the at least one active ingredient is chosen from the group consisting of biocides, insecticides, and fungicides.

In one embodiment, the at least one active ingredient is at least one biocide. Exemplary classes of biocides include, but are not limited to, germicides, bactericides, fungicides, algaeicides, rodenticides, avicides, molluscicides, piscicides, insecticides, acaricides and products to control other arthropods, disinfectants, human hygiene biocidal products, private area and public health disinfectants, veterinary hygiene biocidal products, food and feed area disinfectants, drinking water disinfectants, pest repellants, pest attractants, antifouling products, embalming fluids, taxidermist fluids, and vertebrate control biocides.

Exemplary biocides includes, but are not limited to, neem oil, isothiazolinones, silver oxides, silver salts (e.g., silver halogenide, silver nitrate, silver sulfate, silver carboxylates (e.g., silver acetate, silver benzoate, silver carbonate, silver citrate, silver lactate, silver salicylate)), copper oxides, copper salts (e.g., copper sulfide, copper nitrate, copper carbonate, copper sulfate, copper halogenides, copper carboxylates), zinc oxides, zinc salts (e.g., zinc sulfide, zinc silicate, zinc acetate, zinc chloride, zinc nitrate, zinc sulfate, zinc gulconate, zinc lactate, zinc oxalate, zinc iodate, zinc iodide), iodopopargyl butyl carbamate, aldehydes, formaldehyde condensates, triazines (e.g., 1,3,5-tris-(2-hydroxyethyl-1,3,5-hexahydrotriazine)), dazomet (e.g., 3,5-dimethyl-2H-1,3,5-thiadiazinane-2-thione), glutaraldehyde (e.g., 1,5 Pentanedial), phenolics, carbonic acid esters, surfactant for hypochlorite (commercially available as Accepta 8001 from Accepta™ Advanced Chemical Technologies), bronopol (10%) (such as Accepta 8004 available from Accepta™ Advanced Chemical Technologies), chlorine release tablets, TriChloroisocyanurate (such as Accepta 8005 available from Accepta™ Advanced Chemical Technologies), DiChloroisocyanurate Granules (such as Accepta 8007 available from Accepta™ Advanced Chemical Technologies), Air Hygiene Biocide (such as Accepta 8008 available from Accepta™ Advanced Chemical Technologies),, Multifunctional Chlorine Tablets, DiChloroisocyanurate (such as Accepta 8009 available from Accepta™ Advanced Chemical Technologies), Bio-Dispersant (Oil Fouling) for Cooling Water Systems (such as Accepta 8010 available from Accepta™ Advanced Chemical Technologies), Eco-Friendly Biocide based on Hydrogen Peroxide & Silver (such as Accepta 8101 available from Accepta™ Advanced Chemical Technologies), Hard Surface Cleaner and Surfactant, Hydrogen Peroxide/Silver (such as Accepta 8102 available from Accepta™ Advanced Chemical Technologies), Tablets for Air Conditioning Systems (such as Accepta 8205 available from Accepta™ Advanced Chemical Technologies), Chlorine Dioxide Tablets (such as Accepta 8502, Accepta 8505, and Accepta 8510, each available from Accepta™ Advanced Chemical Technologies Stabilised Chlorine Dioxide in Solution (1000ppm) (such as Accepta 8520 available from Accepta™ Advanced Chemical Technologies), 2 Part Chlorine Dioxide Powder - 0.3% solution in 25L (such as Accepta 8551 available from Accepta™ Advanced Chemical Technologies), 2 Part Chlorine Dioxide Powder - 0.3% solution in 100L (such as Accepta 8552 available from Accepta™ Advanced Chemical Technologies), 2 Part Chlorine Dioxide Powder - 0.3% solution in 1000L (such as Accepta 8553 available from Accepta™ Advanced Chemical Technologies), THPS (such as Accepta 2102 available from Accepta™ Advanced Chemical Technologies), Glutaraldehyde (such as Accepta 2103 available from Accepta™ Advanced Chemical Technologies), Sodium Bromide Solution (40%) (such as Accepta 2104 available from Accepta™ Advanced Chemical Technologies), Isothiazoline (such as Accepta 2113 available from Accepta™ Advanced Chemical Technologies), Biocide for Cooling Water Systems (such as Accepta 2301 available from Accepta™ Advanced Chemical Technologies), Biodegradable Biocide for Cooling Water Systems (such as Accepta 2302 available from Accepta™ Advanced Chemical Technologies), Bio-dispersant for Cooling Water Systems (such as Accepta 2306 available from Accepta™ Advanced Chemical Technologies), 1.5% Isothiazoline, (such as Accepta 2027 available from Accepta™ Advanced Chemical Technologies), DBNPA, 2.2-DiBromo 3-Nitrilo Proprionamide (such as Accepta 2028 available from Accepta™ Advanced Chemical Technologies), Hypochlorite plus Surfactant to Penetrate Biofilms (such as Accepta 2029 available from Accepta™ Advanced Chemical Technologies), 5% Chlorite for Chlorine Dioxide Generators (such as Accepta 2055 available from Accepta™ Advanced Chemical Technologies), 8% Chlorite for Chlorine Dioxide Generators (such as Accepta 2056 available from Accepta™ Advanced Chemical Technologies), 25% Chlorite for Chlorine Dioxide Generators (such as Accepta 2057 available from Accepta™ Advanced Chemical Technologies), Bromine Tablets (BCDMH) (such as Accepta 2073 available from Accepta™ Advanced Chemical Technologies), Biodispersant (such as Accepta 2075 available from Accepta™ Advanced Chemical Technologies), Stabilised Bromine (Activated) (such as Accepta 2078 available from Accepta™ Advanced Chemical Technologies), 3% isothiazolin (such as Accepta 2086 available from Accepta™ Advanced Chemical Technologies), Quaternary Biocide, Ammonium based plus Antifoam (such as Accepta 2087 available from Accepta™ Advanced Chemical Technologies), 0.5% Isothaizoline (such as Accepta 2093 available from Accepta™ Advanced Chemical Technologies), Bacteria Anti-foulant for Marine Cooling Water Systems (such as Accepta 3553 available from Accepta™ Advanced Chemical Technologies), Rapid Dissolving Micro-Chlorine Tablets (such as Accepta 9010 available from Accepta™ Advanced Chemical Technologies), Multifunctional Chlorine Tablets (200g) (such as Accepta 9011 available from Accepta™ Advanced Chemical Technologies), amides (e.g., N(3,4-dichlorophenyl)-N,N-dimethyl urea), carbamates (e.g., methyl-N-benzimidazol-2-methylcarbamate), thiocarbamates, thiocyanates, dibenzamidines, pyridine derivatives, triazoles, thiazoles, isothiazolones, (e.g., 2-methyl-4-isothiazolin-3-one), N-haloalkylthio compounds (e.g., N-dichlorofluoromethylthiophthalimide), a blend of 2-Methyl-4-isothiazolin-3-one (MIT) and 5-Chloro-2-methyl-4-isothiazolin-3-one (CIT) known as CIT/MIT, a combination of CIT/MIT and hydroxymethyl ureide derivatives (such as Acticide® FI(N) and Acticide® FS(N)), a combination of CIT/MIT and formaldehyde (such as Acticide® HF), a combination of CIT/MIT and bronopol monovalent stabilized (such as Acticide® LA), aqueous formulations of MIT, BIT, and hydroxymethyl ureide derivatives (such as Acticide® MBF), aqueous formulations of MIT/BIT Novel CIT (such as Acticide® MBS), sodium nitrate stabilized bivalent metal free aqueous CIT/MIT formulation (such as Acticide® MV), magnesium and copper stabilized aqueous CIT/MIT formulation (such as Acticide® RS), magnesium nitrate stabilized aqueous CIT/MIT formulation (such as Acticide® SPX), and a combination of bacticides and fungicides in aqueous solvent based and powder form (such as Acticide® TBW, Acticide® TBS, and Acticide® TBP). In one embodiment, the at least one active ingredient is Neem oil. In another embodiment, the at least one active ingredient is an isothiazolinone. Exemplary isothiazolin-3-ones include, but are not limited to, 2-methyl-4-isothiazolin-3-one, 2-ethyl 4-isothiazolin-3-one, 2-propyl-4-isothiazolin-3-on, 2-butyl-4-isothiazolin-3-one, 2-amyl-4-isothiazolin-3-one, 5-chloro-2-methyl-4-isothiazolin-3-one, 5-bromo-2-methyl-4-isothiazolin-3-one, 5-iodo-2-methyl-4-isothiazolin-3-one, 5-chloro-2-butyl-4-isothiazolin-3-one, 5-bromo-2-ethyl-isothiazoline-3-one, 5-iodo-2-amyl-4-isothiazolin-3-one, 1,2-benzisothiazolin-3-one, 2-n-octyl-4-isothiazolin-3-one, 4,5-dichloro-2-n-octyl-4-isothiazolin-3-one and other similar analogues and homologues within the genus. Other exemplary biocides are listed in Commission Regulation (EC) No. 1048/2005 of 13 June 2005 from the Official Journal of the European Union.

In another embodiment, the at least one active ingredient is at least one pesticide. Exemplary pesticides include, but are not limited to, (((2-dihydro-5-methyl-3(2H)-oxazolyl)-1-methylethoxy)methoxy)methanol, ([[[(2-dihydro-5-methyl-3(2H)-oxazolyl)-1-methylethoxy]methoxy)methoxy]methanol, 1,1,2,3-tetramethyl butylamine dodecyl benzene sulphonate, 1,12-Di(3-decyl-2-methylimidazolium)dodecane dibromide, 1,2-benzisothiazolin-3-one, 1,2-Benzisothiazolin-3-one, 2-butyl- 1,2-dibromo-2,4-dicyanobutane, 1,3-Bis(2-hydroxyethyl)-2-heptadecenyl imidazolinium chloride, 1,3-dibromo-5,5-dimethylhydantoin, 1,3-dichloro-5,5-dimethylhydantoin, 1,3-Dichloro-5-ethyl-5-methylhydantoin, 1,3-propane diamine dodecyl benzene sulphonate, 1,3-Propanediamine, N-(3-aminopropyl)-N-dodecyl-1,4-bis (bromoacetoxy)-2-butene,1-(2-hydroxyethyl)-2-alkyl-2-imidazoline, alkyl derived from tall oil fatty acids, 1-(2-Hydroxyethyl)-2-heptadecenyl-2-imidazoline, 1-(2-Hydroxyethyl)-2-n-alkyl(C18)-2-imidazoline 1-(3-chloroallyl)-3,5,7-triaza-1-azonia adamantane chloride, 1-(Alkyl* amino)-3-aminopropane *(as in fatty acids of coconut oil), 1-(Alkyl* amino)-3-aminopropane diacetate *(37%C18', 29%C16, 23%C18, 3%C16', 3%C14, 1.5%C18", 1%C14', 1%C12, 0.5%C15), 1-(Alkyl* amino)-3-aminopropane *(47%C12, 18%C14, 10%C18, 9%C10, 8%C16, 8%C8), 1-(Alkyl* amino)-3-aminopropane diacetate *(as in fatty acids of coconut oil), 1-(Alkyl* amino)-3-aminopropane monoacetate *(47%C12, 18%C14, 10%C18, 9%C10, 8%C16, 8%C8), 1-(Alkyl* amino)-3-aminopropane propionate-copper acetate complex 1-(alkyl-amino)-3-aminopropane hydrochloride 1-(alkylamino)-3-aminopropane 1-(alkylamino)-3-carboxymethylaminopropane, 1-Alkyl (C6 to C18) amino-3-aminopropane acetate 1-alkylamino-2-aminopropane monoacetate, alkyl derived from coconut oil fatty acids, 1-Alkylamino-3-aminopropane hydroxyacetate, alkyl derived from coconut oil fatty acids 1-bromo-3-chloro-5,5-dimethyl hydantoin, 1-Heptadecenyl-2-(2-hydroxyethyl)-2-imidazolinium chloride 1-hydroxyethyl-1-benzyl-2-alkyl imidazolinium chloride, 1-Hydroxymethyl-5,5-dimethyl hydantoin 1-Hydroxymethyl-5,7-dichloroindazole, 1-methyl-1-tallow-2-amido ethyl-2-heptadecyl imidazolinium nonemethosulfate, 1-methyl-3,5,7-triaza-1-azonia tricyclo (3.3.1.1.3.7) decane 10,10'-Oxybisphenoxyarsine, 2,2'-(1-methyltrimethylenedioxy)bis-(4-methyl-1,3,2-dioxaborinane), 2,2'-(coco alkyl imino) bis (ethanol) with bis (2-ethylhexyl)phosphate (1:1), 2,2'-(coco alkyl imino) bis (ethanol) with mono (2-ethylhexyl)phosphate (1:1), 2,2'-Dithiobisbenzothiazole ,2,2'-Methylenebis(4,6-dichlorophenol) 2,2'-oxybis (4,4,6-trimethyl-1,3,2-dioxaborinane), 2,2-dibromo-3-nitrilopropionamide 2,2-methylene bis(4,6-dichlorophenol) sodium salt, 2,2-oxybis (4-dodecyl benzene sulfonate), 2,3,5,6-tetrachloro-4-(methylsulfonyl) pyridine, 2,3,5,6-tetrachloro-4-(methylsulfonyl) pyridine, other related 2,3,5-trichloro-4-(propylsulfonyl) pyridine, 2,3,5-trichloro-4-(propylsulfonyl)pyridine, other related 2,4,5-trichlorophenol, 2,4,5-Trichlorophenol salt of 2,6-bis((dimethylamino)methyl)cyclohexanone 2,4,5-trichlorophenol potassium salt, 2,4,5-trichlorophenol, potassium salt, other related 2,4,5-Trichlorophenol sodium salt 2,4,5-trichlorophenol sodium salt, other related 2,4,6-trichlorophenol, 2,4,6-Trichlorophenol sodium salt, 2,4-xylenol 2,6-bis (1-methyl heptadecyl)-p-cresol, 2-((2-amino ethyl) amino) ethanol dodecyl benzene sulphonate, 2-((hydroxymethyl)amino) ethanol, 2-((hydroxymethyl)amino)-2-methyl-1-propanol, 2-(2-(p-(diisobutyl) phenoxy) ethoxy) ethyl dimethyl ammonium chloride, 2-(4-chlorophenylmethyl)-3(2H)-isothiazolone, 2-(decylthio)ethanamine, hydrochloride salt, 2-(p-Hydroxyphenyl)oxoacetohydroximic chloride, 2-(thiocyanomethylthio)benzothiazole with 2'-hydroxyethyl-2,3-dibromopropionate, 2-Alkyl*-1(or 3)-benzyl-1-(2-hydroxyethyl)-2-imidazolinium chloride *(as 1n fatty acids of tall oil), 2-Alkyl*-1-(2-hydroxyethyl)-2-imidazoline phosphate *(100% C13), 2-Alkyl*-1-(4-chlorobutyl)-1-(2-hydroxyethyl)-2-imidazolinium chloride *(100% C6-C17), 2-Alkyl*-1-benzyl-1-(2-hydroxyethyl)-2-imidazolinium nitrate *(100% C11-C17), 2-Alkyl*-2-imidazolinium hydroxide-1-carboxymethyl oxyethyl carboxymethyl, disodium salt of *(as in fatty acids of coconut oil), 2-alkyl-1-benzyl-1-(2-hydroxyethyl)-2-imidazolinium chloride (alkyd 2-bromo-4-hydroxyacetophenone 2-butoxy ethanol phosphate 2-butoxyethanol 2-chloro-4-phenylphenol 2-chloro-4-phenylphenol, potassium salt, 2-chloro-4-phenylphenol, sodium salt, 2-Chlorophenol, 2-ethoxyethanol, 2-Heptadecenyl-2-imidazoline, 2-heptadecyl-1-hydroxyethyl-imidazolinium hydrochloride, 2-heptadecyl-1-methyl-1-(2-stearoyl amide) ethyl) imidazoliniummethyl sulfate, 2-heptyl-2-imidazolinium hydroxide-1-carboxy methyloxy ethyl-1-carboxy methyl, disodium salt, 2-Lauryl isoquinolinium bromide, 2-Mercaptobenzothiazole, 2-Mercaptobenzothiazole, 1-(hydroxyethyl)pyridinium salt, 2-Mercaptobenzothiazole, ferric salt, 2-Mercaptobenzothiazole, manganese salt, 2-Mercaptobenzothiazole, monoethanolammonium salt, 2-Mercaptobenzothiazole, potassium salt, 2-Mercaptobenzothiazole, sodium salt, 2-Mercaptobenzothiazole, zinc salt, 2-Methyl-3(2H)-isothiazolone, calcium chloride complex, 2-methyl-4,5-trimethylene-4-isothiazolin-3-one 2-methyl-4-isothiazolin-3-one 2-Nitro-2-ethyl-1,3-propanediol, 2-Nitro-2-methyl-1,3-propanediol 2-tert-Amylphenol 3(2H)-chloro-2-methyl-3(2H)-isothiazolone, calcium chloride complex 3,3,4,4-tetrachloro tetrahydro thiophene, 1,1-dioxide 3,3,4,4-tetrachlorotetrahydrothiophene, 1,1-dioxide, other related 3,3,4-Trimethyloxazolidine mixt. with 4,4-dimethyloxazolidine 3,4,4-trimethyl oxazolidine 3,4,5-tribromo salicylanilide, other related 3,4-Dichloro-5-isothiazolecarboxylic acid, 3,5-dibromosalicylanilide, 3-(Hydroxymercuri)-4-nitro-o-phenol, sodium salt, 3-(trimethoxysilyl) propyl dimethyl octadecyl ammonium chloride, 3-Bromo-1-chloro-5,5-dimethylhydantoin, 3-chloro-2-biphenylol, sodium salt, 4 & 6 - chloro-2-phenylphenol, 4 & 6 - chloro-2-phenylphenol, potassium salts, mixed 4 & 6 - chloro-2-phenylphenol, sodium salts, mixed 4 & 6-chloro-2-phenylphenol, other related 4 or 6-chloro-2-phenylphenol 4 or 6-chloro-2-phenylphenol, diethanolamine salt, 4'-hydroxy-m-phenoxy benzoic acid 4,4-(2-ethyl-2-nitrotrimethylene) dimorpholine, 4,4-dimethyl oxazolidine, 4,4-dimethyl-2-oxazolidinone, 4,5-dibromosalicylanilide 4,5-dichloro-2-n-octyl-3(2H)-isothiazolone, 4,6-dichloro-2-phenylphenol, 4,6-dichloro-2-phenylphenol, potassium salt, 4-(2-nitrobutyl) morpholine 4-chloro-2-cyclopentyl phenol, potassium salt 4-chloro-2-cyclopentylphenol, 4-chloro-2-n-octyl-3(2H)-isothiazolone, 4-chloro-2-phenylphenol, 4-chloro-2-phenylphenol, potassium salt, 4-chloro-2-phenylphenol, sodium salt, 4-chloro-cyclopentylphenol, sodium salt, 5,4-dibromosalicylanilide, 5-Bromo-2-methyl-5-nitro-1,3-dioxane 5-chloro-2-((4-chlorophenyl)methyl)-3(2H)-isothiazolone, compound with 2-((4-chlorophenyl)methyl)-3(2h)-isothiazolone (1:1), 5-chloro-2-(4-chlorophenylmethyl)-3(2H)-isothiazolone, 5-Chloro-2-mercaptobenzothiazole, lauryl pyridinium salt 5-Chloro-2-mercaptobenzothiazole, zinc salt, 5-chloro-2-methyl-4-isothiazolin-3-one, 5-chloro-2-methyl-4-isothiazolin-3-one calcium chloride with 2-methyl-4-isothazolin-3-one calcium chloride, 5-hydroxy methoxy methyl-1-aza-3,7-dioxabicyclo (3.3.0) octane 5-hydroxymethyl-1-aza-3,7-dioxabicyclo (3.3.0) octane 5-hydroxypoly (methyleneoxy) (74%C2, 21%C3, 4%C4, 1%C5) methyl-1-aza-3,7-dioxabicyclo (3.3.0), octane 6-chloro-2-phenylphenol, 6-chloro-2-phenylphenol, potassium salt, 6-tert-Butyl-4-chloro-m-cresol, 8-Hydroxyquinoline, 8-Hydroxyquinoline benzoate, 8-Hydroxyquinoline citrate, 8-Hydroxyquinoline sulfate a-(p-nonylphenyl)-omega-hydroxypoly (oxyethylene) with an average of 4.2 moles of ethylene oxide.the nonyl group is a propylene Abietylamine Acetalized polyvinyl alcohol complexed with iodine Acetic acid Acetic acid, sodium salt, (2:1) Acylamido alkylbenzyl dimethyl ammonium chloride, Alcohols, C4-C12, normal Alcohols, C8-10 Alkenyl (75%C18, 25%C16) dimethyl amine acetate, Alkenyl (90%C18, 10%C16) dimethyl ethyl ammonium bromide, Alkenyl* amine o-phenylphenate *(C8-C18), Alkenyl* dimethyl ammonium chloride *(75% C18', 25% C16'), Alkenyl* dimethyl ethyl ammonium bromide *(90%C18', 10%C16'), Alkenyl* dimethyl ethyl ammonium chloride *(75% C18', 25% C16'), Alkenyl* dimethyl ethyl ammonium chloride *(90% C18', 10% C16') Alkyl (29%C14, 29%C13, 21%C12, 21%C15) poly(oxypropylene) poly (oxyethylene) - iodine complex, Alkyl (46%C12, 24%C14, 10%C16, 8%C10, 7%C8, 5%C18) aminobetaine, Alkyl (47%C12, 18%C14, 10%C18, 10%C16, 15%C8-C10) dimethylbenzyl ammonium chloride, Alkyl (50%C12, 30%C14, 17%C16, 3%C18) dimethyl dichlorobenzyl ammonium chloride, Alkyl (50%C12, 30%C14, 17%C16, 3%C18) dimethylbenzyl ammonium chloride, Alkyl (50%C12, 30%C14, 17%C16, 3%C18) dimethylethylbenzyl ammonium chloride, Alkyl (50%C14, 40%C12, 10%C16) dimethylbenzyl ammonium saccharinate, Alkyl (50%C14, 40%C12, 10%C16) dimethylbenzyl ammonium chloride, Alkyl (53%C12, 19%C14, 8.5%C16, 7%C8, 6.5%C10, 6%C18)-1,3-propane diamine, Alkyl (58%C14, 28%C16, 14%C12) dimethylbenzyl ammonium chloride, Alkyl (58%C18, 40%C16, 1%C14, 1%C12) trimethyl ammonium chloride, Alkyl (60%C14, 25%C12, 15%C16) dimethylbenzyl ammonium chloride, Alkyl (60%C14, 30%C16, 5%C12, 5%C18) dimethylbenzyl ammonium chloride, Alkyl (61%C12, 23%C14, 11%C16, 3%C10, 2%C8) dimethylbenzyl ammonium chloride, Alkyl (61%C12, 23%C14, 11%C16, 5%C18) dimethyl benzyl ammonium chloride, Alkyl (61%C12, 23%C14, 11%C16, 5%C8,C10,C18) dimethylbenzyl ammonium chloride, Alkyl (61%C12,23%C14,11 %C16,2.5%C8 & C10,2.5%C18) dimethyl benzyl ammonium chloride, Alkyl (65%C12, 25%C14, 10%C16) dimethylbenzyl ammonium chloride, Alkyl (67%C12, 25%C14, 7%C16, 1%C8,C1 0,C18) dimethylbenzyl ammonium chloride Alkyl (68% C12, 32% C14) dimethyl dimethylbenzyl ammonium chloride, Alkyl (68%C12, 32%C14) dimethylethylbenzyl ammonium chloride, Alkyl (70%C18, 27%C16, 3%C14) trimethyl ammonium chloride, Alkyl (90%C14, 5%C12, 5%C16) dimethyl dichlorobenzyl ammonium chloride, Alkyl (90%C14, 5%C12, 5%C16) dimethylbenzyl ammonium chloride, Alkyl (90%C14, 5%C12, 5%C16) dimethyl ethyl ammonium bromide, Alkyl (93%C14, 4%C12, 3%C16) dimethylbenzyl ammonium chloride, Alkyl (95%C14, 3%C12, 2%C16) dimethyl benzyl ammonium chloride dehydrate, Alkyl (95%C14, 3%C12, 2%C16) dimethyl benzyl ammonium chloride monohydrate, Alkyl (95%C14, 3%C12, 2%C16) dimethyl benzyl ammonium chloride, Alkyl (98%C12, 2%C14) dimethyl 1-naphthylmethyl ammonium chloride, Alkyl (C10-16) dimethyl amine oxide, Alkyl (C10-C14) benzene sulfonic acid, calcium salt, Alkyl (C12-C15)-poly(oxypropylene) poly(oxyethylene) - iodine complex, Alkyl (C14, C12, C16) dimethyl benzyl ammonium chloride, Alkyl (C8-C18) bis (2-hydroxyethyl) benzyl ammonium chloride, Alkyl benzene sulfonic acid, Alkyl dimethyl amine, Alkyl dimethyl cumenyl ammonium chloride, Alkyl dimethyl ethyl benzyl ammonium cyclohexyl sulphonate, Alkyl dimethyl ethylbenzyl ammonium cyclohexyl sulfamate, Alkyl dimethyl isopropyl benzyl ammonium chloride, Alkyl imidazoline monocarboxylate, monosodium salt, Alkyl methyl isoquinolinium chloride, Alkyl monoethyoxyethanol, diethoxyethyl benzyl ammonium chloride, Alkyl trimethyl ammonium bromides, Alkyl* (ethyl cycloimidinium) 3-hydroxy-3-ethyl sodium alcoholate, 2-methyl sodium carboxylate-tridecyl polyoxyethylene ethanol, Alkyl* benzyl trimethyl ammonium chloride *(56%C12', 27%C11, 9%C13', 4%C10', 3%C14', 1%C15'), Alkyl* benzyl trimethyl ammonium chloride *(50%C12, 20%C13, 10%C14, 10%C11, 10%C10), Alkyl* bis(2-hydroxyethyl) amine acetate *(65% C18, 30% C16, 5% C14), Alkyl* dihydroxyethyl amine oxide *(50%C12, 27%C14, 10%C16, 8%C10, 5%C8), Alkyl* dimethyl 3,4-dichlorobenzyl ammonium chloride *(90%C14, 5%C16, 5%C12), Alkyl* dimethyl 3,4-dichlorobenzyl ammonium chloride *(60%C12, 25%C14, 15%C16), Alkyl* dimethyl 3,4-dichlorobenzyl ammonium chloride *(55%C14, 23%C12, 20%C16, 2%C18), Alkyl* dimethyl 3,4-dichlorobenzyl ammonium chloride *(65%C12, 22%C14, 10%C16, 3%C18), Alkyl* dimethyl 3,4-dichlorobenzyl ammonium chloride *(50%C14, 40%C12, 10%C16), Alkyl* dimethyl 3,4-dichlorobenzyl ammonium chloride *(63% C12, 23% C14, 14% C16), Alkyl* dimethyl 3,4-dichlorobenzyl ammonium chloride *(68% C12, 27% C14, 5%C16), Alkyl* dimethyl 3,4-dichlorobenzyl ammonium chloride *(C12 65%, C14 25%, C16 10%), Alkyl* dimethyl 3,4-dichlorobenzyl ammonium chloride *(75% C12, 25% C14), Alkyl* dimethyl 3,4-dichlorobenzyl ammonium chloride *(60% C14, 30% C16, 10% C12), Alkyl* dimethyl 3,4-dichlorobenzyl ammonium chloride *(42% C12, 27% C14, 13% C16, 18% C8-C18), Alkyl* dimethyl 3,4-dichlorobenzyl ammonium chloride polyethoxypolypropoxypolyethoxyethanol - iodine complex *(50%C12, 30%C14, 1 Alkyl* dimethyl ammonium bromide *(50% C12, 30% C14, 17% C16, 3% C18), Alkyl* dimethyl ammonium bromide *(67% C16, 30% C14, 3% C12-C18), Alkyl* dimethyl benzyl ammonium chloride *(60%C14, 30%C16, 5%C18, 5%C12), Alkyl* dimethyl benzyl ammonium chloride *(50%C12, 30%C14, 17%C16, 3%C18), Alkyl* dimethyl benzyl ammonium chloride *(100% C16), Alkyl* dimethyl benzyl ammonium chloride *(55%C16, 20%C14, 20%C12, 5%C18), Alkyl* dimethyl benzyl ammonium chloride *(67%C12, 25%C14, 7%C16, 1%C18), Alkyl* dimethyl benzyl ammonium chloride *(61 % C12, 23% C14, 11 % C16, 5% C18), Alkyl* dimethyl benzyl ammonium chloride *(41%C14, 28%C12, 19%C18, 12%C16), Alkyl* dimethyl benzyl ammonium bentonite *(as in fatty acids of tallow), Alkyl* dimethyl benzyl ammonium chloride *(55% C16, 27% C12, 16% C14, 2% C18), Alkyl* dimethyl benzyl ammonium chloride *(95% C16, 5% C18), Alkyl* dimethyl benzyl ammonium chloride *(100% C18), Alkyl* dimethyl benzyl ammonium ion alkyl** amine *(C12, C14, C16) **(C10, C12, C14, C16), Alkyl* dimethyl benzyl ammonium chloride *(65%C12, 25%C14, 10%C16), Alkyl* dimethyl benzyl ammonium chloride *(93%C14, 4%C12, 3%C16), Alkyl* dimethyl benzyl ammonium chloride *(47%C12, 18%C14, 15%(C5-C15), 10%C18, 10%C16), Alkyl* dimethyl benzyl ammonium chloride *(58%C14, 28%C16, 14%C12), Alkyl* dimethyl benzyl ammonium chloride *(60%C14, 25%C12, 15%C16), Alkyl* dimethyl benzyl ammonium chloride *(60% C14, 30% C16, 10% C12), Alkyl* dimethyl benzyl ammonium chloride *(65%C12, 23%C14, 12%C16), Alkyl* dimethyl benzyl ammonium chloride *(67%C12, 25%C14, 7%C16, 1%C8, C10, and C18), Alkyl* dimethyl benzyl ammonium chloride *(67%C12, 27%C14, 6%C16), Alkyl* dimethyl benzyl ammonium chloride *(68% C12, 25% C14, 7% C16), Alkyl* dimethyl benzyl ammonium chloride *(90%C14, 5%C12, 5%C16), Alkyl* dimethyl benzyl ammonium chloride *(as in fatty acids of coconut oil), Alkyl* dimethyl benzyl ammonium chloride *(C8-18), Alkyl* dimethyl benzyl ammonium chloride *(61%C12, 23%C14, 11%C16, 3%C10, 2%C18), Alkyl* dimethyl benzyl ammonium chloride *(40%C12, 40%C14, 20%C16), Alkyl* dimethyl benzyl ammonium dichloroisocyanurate *(60%C14, 30%C16, 6% C12, 4% C18), Alkyl* dimethyl betaines *(46%C12, 24%C14, 10%C16, 8%C10, 7%C8, 5%C18), Alkyl* dimethyl ethyl ammonium bromide *(85% C16, 15% C18), Alkyl* dimethyl ethyl ammonium bromide *(mixed alkyl and alkenyl groups as in fatty acids of soybean oil), Alkyl* dimethyl ethylbenzyl ammonium chloride *(60%C14, 30%C16, 5%C12, 5%C18), Alkyl* dimethyl furfuryl ammonium chloride *(65% C18, 30% C16, 5% C14), Alkyl* dimethyl isopropylbenzyl ammonium chloride *(60%C14, 30%C16, 5%C12, 5%C18), Alkyl* dimethyl trichlorobenzyl, Ammonium chloride *(50%C14, 40%C12, 10%C16),, Alkyl* dodecylbenzyl dimethyl, Ammonium chloride *(67% C18, 33% C16), Alkyl* monoethoxyethanol diethoxyethanol benzyl, Ammonium chloride *(48%C12, 17%C14, 11%C18, 9%C16, 8%C8, 7%C10), Alkyl* N,N-bis(2-hydroxyethyl)amine *(100% C8-C18), Alkyl* poly(oxypropylene)poly(oxyethylene)-iodine complex *(43%C10, 30%C14, 12%C12, 10%C16, 5%C18), Alkyl* sodium benzene sulfonate *(56%C11, 33%C12, 7%C10, 4%C13), Alkyl* tolyl methyl trimethyl, Ammonium chloride *(100% C9-C15), Alkyl* tributyl phosphonium chloride *(95%C12, 3%C14, 2%C10), Alkyl* trimethyl, Ammonium bromide *(95%C14, 5%C16), Alkyl* trimethyl, Ammonium chloride *derived from cottonseed oil, Alkyl* trimethyl, Ammonium chloride *(90%C18, 10%C16), Alkyl* trimethyl, Ammonium chloride *(70%C18, 27%C16, 3%C14), Alkyl* trimethyl, Ammonium chloride *(as in fatty, Acids of coconut oil), Alkyl* trimethyl, Ammonium chloride *(70% C12, 30% C14), Alkyl* trimethyl, Ammonium chloride *(alkyl, As in fatty, Acids of soybean oil), Alkyl* trimethyl, Ammonium chloride *(58% C18, 40% C16, 1% C14, 1% C12), Alkyl* triphenyl phosphonium cloride *(95%C12, 3%C14, 2%C10), Alkyl*-1-(2-aminoethyl)-2-imidazoline, Acetate - nickel sulfate complex, Alkyl*-5-hydroxy-4-oxo-2(4H)-pyranylmethyl dimethyl, Ammonium chloride *(49% C12, 17% C14, 17% C10, 10% C18, 9% C16, 8% C8), Alkyl*-diamine monobenzoate *(as in fatty, Acids of coconut oil), Alkyl*-N,N-bis(2-hydroxyethyl)methyl, Ammonium chloride *(53% C12, 19% C14, 8.5% C16, 7.0% C8, 6.5% C10, 6.0% C18), Alkyl*-N,N-di(hydroxyethyl)amine *(as in fatty, Acids of soybean oil), Alkyl-1,3-propylene diamine, Acetate,, Alkyl derived from coconut oil fatty, Acids, Alkyl-1,3-propylene diamine, Adipate,, Alkyl derived from coconut oil fatty, Acids, Alkyl-1,3-propylene diamine,, Alkyl derived from coconut oil fatty, Acids, Alkylamine, Acetate, Alkylamine hydrochloride, Alkyl derived from coconut oil fatty, Acids, Alkylaryl sulfonates, Alkylaryl sulfonates,, Amine salts, Alkyldimethylbenzyl, Ammonium chloride, Alkyldimethylethylbenzylammonium chloride, Alkyltrimethyl, Ammonium chloride, Alpha-alkyl (43%C10, 30%C14, 12%C12, 10%C16, 5%C18) poly (oxyethylene) poly (oxypropylene) - 12 complex,, Alpha-Alkyl*-omega-hydroxypoly(oxyethylene)ethanol-iodine complex *(100% C12-C15), Alpha-terpineol, Amines, C10-16-alkyldimethyl, N-oxides, Ammonium, Acetate, Ammonium carbonate, Ammonium hydroxide, Ammonium hydroxide - C8 fatty acid silver complex, Ammonium lauryl sulfate, Ammonium nitrate Ammonium nitrite, Ammonium oxalate, Ammonium propionate, Arquad DMCB (16908+047501), Benax 2-A-1 (079002 + 079003) Benzene, 1,1'-oxybis-tetrapropylene derivatives, sulfonated, sodium, Benzyl((dodecylcarbamoyl)methyl)dimethyl ammonium chloride, Benzyl-C12-14-alkyldimethyl quaternary ammonium compounds, Beta-bromo-beta-nitrostyrene, Bioban p-1487, Biobor JF, Biphenyl, Bis (tributyltin) adipate, Bis (tributyltin) sulfone, Bis (trichloromethyl) sulfone, Bis(tributyltin) dodecenylsuccinate, Bis(tributyltin) salicylate, Bis(tributyltin) succinate, Bis(tributyltin) sulfosalicylate, Bis(tripropyltin) oxide, Bromine, Bromine chloride, Bronopol, BTC 2125-m, Butoxy polypropoxy polyethoxy ethanol - iodine complex, Butyl paraben, Butylamine dodecyl, Benzene sulfonate C12-16-alkyldimethyl amines Calcium, Carbonate, Calcium, Carbonate, treated, Calcium, Chlorate, Calcium, Chloride, Calcium ethylenebis(dithiocarbamate), Calcium hydroxide, Calcium hypochlorite-dodecylbenzene sulfonate, Complex, Calcium nitrite, Calcium oxytetracycline, Calcium phosphate, tribasic, Calcium silicate, Camphor, Camphor oil, Capric acid, Caprylic acid, Cetyl dimethyl ethyl ammonium bromide, Cetyl pyridinium, Chloride, Cetyl pyridium bromide, Cetyl trimethyl ammonium bromide, Cetyl trimethyl ammonium, Chloride, Chloramine B, Chloramine T, Chlorbisan, Chlorhexidine, Chlorhexidine diacetate, Chlorhexidine digluconate, Chlorhexidine dihydrochloride, Chlorhexidine gluconate,, Chlorine,, Chlorine dioxide, Chlorite, Clay,, Chloroethylene bisthiocyanate,, Chlorophyllin,, Chloroxylenol, Chromated zinc, Chloride,, Citric acid,, Citric acid ammonium salt,, Coal tar phenols, Coconut imidazoline, sodium, Carboxylate,, Condensation product of mixed, Cresols, formaldehyde and sulfanilic acid,, Copper 2-pyridinethio-1-oxide,, Copper 8-quinolinoleate,, Copper ammonium, Complex (acetate/carbonate), Copper hydroxide,, Copper pentachlorophenate,, Copper zeolite,, Cuprous thiocyanate, Cyanuric acid,, Cyanuric acid, Chloride,, Cyanuric acid monosodium salt, Decyl phenoxy benzene disulfonic acid disodium salt, Decyl phenoxy benzene disulfonic acid, Decyl-N-methyl-N-(3-trimethoxysilyl)propyl)-1-decanaminium chloride, Dehydroabietylamine, Dehydroabietylamine acetate, Dehydroabietylamine pentachlorophenate, Device (swimming pool algaecide,bacteriacide, molluscicide) no guarantee required Di(alkyl* oxypropyl)dimethyl ammonium chloride *(60%C8, 40%C10), Di(phenylmercuric) dodecenyl succinate Dialkyl (85%C18, 15%C16), dimethyl ammonium chloride, Dialkyl dimethyl ammonium polynaphthyl amine, Dialkyl* dimethyl ammonium bromide *(50% C12, 30% C14, 17% C16, 3% C18), Dialkyl* dimethyl ammonium chloride *(70%C18, 26%C16, 4%C14), Dialkyl* dimethyl ammonium chloride *(47%C12, 18%C14, 10%C18, 9%C10, 8%C16, 8%C8), Dialkyl* dimethyl ammonium chloride *(50%C12, 30%C14, 20%C16), Dialkyl* dimethyl ammonium chloride *(50%C12, 20%C14, 15%C10, 10%C16, 5%C18), Dialkyl* dimethyl ammonium chloride *(48% C12, 17% C14, 10% C18, 9% C16, 8% C8, 7% C10, 1% C6), Dialkyl* dimethyl ammonium chloride *(74%C18, 26%C16), Dialkyl* methyl benzyl ammonium chloride *(60% C14, 30% C16, 5% C18, 5% C12), Diammonium ethylenebis(dithiocarbamate), Diammonium phosphate, Dibenzyldimethylammonium chloride, Dichloro-1,2-dithiol-3-one Dichloro-s-triazinetrione, Dichloroisocyanuric acid potassium salt, Dichlorophene Dicoco dimonium chloride, Dicyandiamide, Didecyl dimethyl ammonium bromide, Didecyl dimethyl ammonium carbonate and didecyl dimethyl ammonium bicarbonate, Didecyl dimethyl ammonium chloride, Didecyl methyl benzyl ammonium chloride, Diethanolamine dodecylbenzenesulfonate, Diethanolamine myristate - iodine complex, Dihydro abietylamine acetate, Diiodomethyl p-chlorophenyl sulfone, Diiodomethyl p-tolyl sulfone, Diisobutyl cresoxy ethoxy ethyl dimethyl benzyl ammonium chloride- ortho-phenylphenol ethoxy nonyl phenol complex, Diisobutyl cresoxyethoxyethyl dimethyl benzyl ammonium chloride, Diisopropyl cresol, Diisopropylamine dodecyl benzene sulphonate, Dilauryl dimethyl ammonium bromide, Dimethoxane, Dimethyl alkyl tertiary amines, Dimethyl dicoco ammonium chloride, Dimethyl-1-(hydroxymethyl)pyrazole Dimethylalmine propylamine tridecyl benzene sulfonate Dimethylamine dodecyl benzene sulfonate Dimethylol dimethyl hydantoin Dimethyltetradecyl [3-(trimethoxysilyl)propyl]ammonium chloride, Dinitro cresol, Dioctyl dimethyl ammonium chloride, Diphenylstibene 2-ethylhexanoate, Dipropylene glycol, Dipyrithione, Disodium 2,2'-methylenebis(3,4,6-trichlorophenate) Disodium 2,2'-methylenebis(4-chlorophenate), Disodium 2,2'-oxybis(4-dodecylbenzene)sulphonate, Disodium 4-dodecyl-2,4'-oxydibenzene sulfonate Disodium cyanodithioimido carbonate, Disodium mono ethanolamine phosphate DNOC sodium salt, Dodecyl di(2-hydroxyethyl) benzyl ammonium chloride, Dodecyl dihydroxy ethyl benzyl ammonium chloride, Dodecyl dimethyl 2,4,5-trimethylbenzyl ammonium chloride, Dodecyl dimethyl ammonium chloride, Dodecyl dimethyl benzyl ammonium chloride, Dodecyl dimethyl benzyl ammonium bromide, Dodecyl dimethyl benzyl ammonium cyclopentanecarboxylate, Dodecyl dimethyl trichlorobenzyl ammonium chloride, Dodecyl guanidine hydrochloride, Dodecylamine salicylate, Dodecylbenzene sulfonic acid, Dodecylbenzene sulfonic acid amine salt, Dodecylbenzene sulfonic acid calcium salt, Dodecylbenzenesulfonic acid-iodine complex, Dodecylbenzyl alkyl (70%C12, 30%C14) dimethyl ammonium chloride, Dodecylbenzyl octadecyl dimethyl ammonium chloride, Dodecylbenzyl trimethyl ammonium chloride, Dodine, Epichlorohydrin, Erythromycin phosphate, Ethanolamine, Ethyl alcohol, Ethyl dimethyl stearyl ammonium bromide, Ethyl paraben, Ethyl-2-heptadecenyl-1-(2-hydroxyethyl)-2-imidazolinium bromide, Ethylene diamine dodecyl benzene sulphonate, Ethylene oxide - alkylated cresol condensate, Ethylhexanoate, Ethylmercury pentachlorophenate Fatty acid* - silver complex *(100% C8), Fenaminosulf, Fenticlor, Ferric nitroso dimethyl dithiocarbamate, Flumequine, Formaldehyde, Gentian violet, Glutaraldehyde, Glycolic acid, Grotan (Hexahydro-1,3,5-tris (2-hydroxyethyl)-s-triazine), Gurjun balsam oil, Heptadecenyl imidazolinium chloride, Heptadecyl hydroxyethyl imidazoline phosphate, Heptadecyl hydroxyethyl imidazoline, Hexachlorobenzene, Hexachlorophene, Hexachlorophene sodium salt, Hexahydro-1,3,5-tris(2-hydroxypropyl)-s-triazine, Hexamethylenetetramine cis-chloroallyl chloride, Hexylene glycol, Homosalate Hydrogen peroxide, Hydroxymercuri-o-nitrophenol Hydroxymercuri-o-nitrophenol sodium salt, Hydroxymercurichlorophenols, Hydroxymercuriphenyl ammonium triethanolamine, Hydroxymercury cresol, Hypochlorite salts, Hypochlorous acid, Imidazoline acetate, Inorganic phosphate, Iodine, Isopropyl 8-((((hydroxymethyl)amino)-2-napthalenyl)sulfonyl)salicylate hydrogen chloride, Isopropyl alcohol, Isopropyl cresol, Isopropyl phenol Isopropylamine dodecylbenzene sulphonate, Isopropylamine methyl naphthalene sulphonate, Isothan Q, Kaolin, Kasugamycin, Kathon 886 (kathon biocide), Lactoxymercuriphenyl ammonium lactate, Lauryl polyoxyethylene sulfate, Lauryl sulfate salts, Lime, Lithium carbonate, Lithium hydroxide, Lithium hypochlorite, Magnesium carbonate, Magnesium chlorate, Magnesium chloride, Magnesium chloride hexahydrate, Magnesium lauryl sulfate, Magnesium nitrate, Magnesium oxide, Magnesium pyrithione, Magnesium silicate, Malic acid, Mercuric dimethyl dithiocarbamate, Mercury and inorganic mercury compounds, Meta-cresol, Metallic silver, Metam potassium, Metam-sodium, Methanol, Methyl alkyl* benzyl trimethyl ammonium chloride *(C9H19 - C15H31), Methyl benzethonium chloride, Methyl dodecyl benzyl trimethyl ammonium chloride, Methyl dodecyl benzyl trimethyl ammonium chloride 80% and methyl, Methyl dodecyl trimethyl ammonium chloride Methyl dodecyl xylene bis (trimethyl ammonium chloride), Methyl paraben, Methyl paraben calcium salt, Methyl paraben sodium salt, Methylene bis(thiocyanate), Methylenebis(thiocyanate) with TBTO, Metronidazole Mixed fatty alkyl* diamines *(42%C12, 26%C18, 15%C14, 8%C16, 5%C10, 4%C8), Mono (trichloro) tetra (monopotassium dichloro) penta-s-triazine, Mono- and di- potassium salts of phosphorous acid, Monoethanolamine dodecylbenzenesulfonate ,Monoethanolamine laurate, myristate, and related compounds, Monotributyltin salicylate, Morpholine, Morpholine dodecyl benzene sulphonate, Morpholine ester of abeitic and oleic acids, Morpholine poly ethoxy ethanol, Morpholinomethyl dimethyldithiocarbamate, N,N'''-1,6-Hexanediylbisguanidine, homopolymer, hydrochloride N,N-bis (2-hydroxy ethyl) octanamide N,N-bis-(2-(omega-hydroxypoly(oxyethylene) ethyl) alkylamine, alkyl derived from coconut oil fatty acids N,N-bis-(2-hydroxy ethyl)-cis-9-octadecenamide N,N-bis-(2-hydroxyethyl) alkyl amine N,N-dimethyl-1,3-propane diamine dodecyl benzene sulfonate N-((1-Alkyl* ethyl)-1,3-propanediamine) *(as in fatty acids of cottonseed oil), N-(1-Methyl alkyl*) dimethylamine *(66% C12, 34% C14) N-(2-Ethyl alkyl*) dimethylamine *(62.6% C14, 35.8% C16, 1.6% C12), N-(2-hydroxy propyl) octanamide N-(2-hydroxyethyl) ethylene diamine triacetic acid,trisodium salt, N-(3,4-dichlorobenzyl)-N-dodecyl-N,N-dimethyl ammonium chloride, N-alkyl (soza)-N-methyl morpholinium sulfate N-Alkyl* dimethyl benzyl ammonium chloride *(57%C12, 18%C14, 8%C16, 6%C10-C18, 5%C8), N-Alkyl* dimethylamine *(76% C16, 12% C14, 10.5% C18, 1.5% C8-C12), N-Alkyl* dimethylamine *(90% C18, 8.5%C16, 1.5%C17), N-Alkyl-1,3-propylene diamine monobenzoate, alkyl derived from coconut oil fatty acids, N-Cetyl-N-ethyl morpholinium ethyl sulfate, N-Dialkyl (60%C14, 30%C16, 5%C12, 5%C18) methyl benzyl ammonium chloride, N-Dodecylguanidine terephthalate N-isononyl-N,N-dimethyl decanaminium chloride, N-methyl-N-oleoyltaurine sodium salt, N-octadecenyl-1,3-propane diamine monogluconate, N-Polyethoxylated oleylamine, N-Polyoxyethylated stearylamine, Naphthalene sulfonic acid/parametaldehyde condensate ammonium salt, Naphthalene-formaldehyde condensate, sulfonic acid, metallic salts, Neomycin sulfate, Nitrapyrin, Nitrapyrin other related Nonyl phenoxy poly (oxyethylene) amine sulfonate ethanol - iodine, Nonyl phenoxy polyoxyethylene ethanol-iodine complex Nuosept 95 (Use code nos., 107001, 107002 and 107003), o-(Chloromercuri)phenol, o-(Hydroxymercuri)benzoic acid, cyclic anhydride o-cresol o-Phenylphenol, alkyl* amine - copper salt of *(100% C8-C18) o-Phenylphenol, alkyl* amine - zinc salt of *(100% C18), o-Phenylphenol, tetradecylamine salt, Octadecyldimethyl trihydroxysilyl propyl ammonium chloride, Octhilinone, Octyl decyl dimethyl ammonium chloride, Octyl dodecyl dimethyl ammonium chloride, Octylphenoxy polyethoxy ethanol - iodine complex, Octylphenoxyethoxyethyl dimethyl 4-chlorobenzyl ammonium chloride, Oil of hardwood, Oil of pine tar, Oleic acid triethanolamine salt, Oleyl dimethyl ethyl ammonium bromide, Omadine, TBAO, Ortho-benzyl-para-chlorophenol, Ortho-benzyl-para-chlorophenol potassium salt, Ortho-benzyl-para-chlorophenol sodium salt, Ortho-phenylphenol Ortho-phenylphenol ammonium salt, Ortho-phenylphenol potassium salt, Ortho-phenylphenol sodium salt, Other chlorinated pyridines of Dowacil A-40 Oxalic acid Oxazolidine E Oxolinic acid Oxydiethylene bis (alkyl dimethyl ammonium chloride) alkyl derived from coconut oil fatty acids, Oxytetracycline, Oxytetracycline hydrochloride, Ozone p-cresol p-Cresyl acetate p-tert-Octylphenoxyethoxyethyl dimethyl benzyl ammonium mercuric chloride, Para-chloro-meta-cresol, para-Chloro-ortho-cresol, Para-tert-amylphenol, Para-tert-amylphenol potassium salt, Para-tertbutylphenol, Paraformaldehyde PCP, PCP, other related PCP, potassium salt PCP, sodium salt PCP, sodium salt, other related Pentachlorophenol, zinc salt Pentachlorophenol, zinc salt of alkyl*-N-propanediamine *(100% C16-C18), Pentachlorophenyl laurate, Peroxyacetic acid, Phenol, Phenols, Phenyl 2-trimethylammonium ethanesulfonate methyl sulfate, Phenyl mercury lauryl mercaptide, Phenylmercuric 2-ethylhexanoate, Phenylmercuric borate, Phenylmercuric carbonate, Phenylmercuric chloride, Phenylmercuric dimethyldithiocarbamate, Phenylmercuric formamide, Phenylmercuric hydroxide, Phenylmercuric lactate, Phenylmercuric monoethanolammonium acetate, Phenylmercuric monoethanolammonium lactate, Phenylmercuric nitrate, Phenylmercuric octanoate, Phenylmercuric oleate, Phenylmercuric propionate, Phenylmercuric salicylate, Phenylmercuric thiocyanate, Phenylmercuritriethanolammonium lactate, Phenylmercury 8-hydroxyquinolinolate, Phenylmercury benzoate, Phosphoric acid, Phosphoric acid, disodium salt, dihydrate Phosphoric acid monopotassium salt, Phosphorous acid ,Phthalaldehyde, Pine soap, Pine tar, Pine tar oil PMA, PMA, other related PMAA Poly (methylmethacrylate-co-tributyltin methacrylate), Poly oxyethylene ethanol monoesters of 5- (and 6-) carboxy-4-hexyl-2-cyclohexene-1-octanoic acid, complex with iodine, Poly propoxy poly ethoxy ethanol, I2 complex Poly(oxyethylene) (dimethylimino) ethylene (dimethylimino) ethylene dichloride, Poly(vinyl/divinylbenzyltrimethylammoniumdiiodochloride/bromide/iodide), Polyacrylonitrile-copper ion-quarternary ammonium compound Polybromide form of trimethylbenzyl ammonium resin, Polychlorophenols and their sodium salts, Polyethoxy polypropoxy polyethoxy ethanol - iodine complex, Polyethoxypolypropoxypolyethoxyethanol-n-alkyl (54% C12, 18% C14, 9% C16, 9% C18, 5% C10, 5% C8), di(beta-hydroxyethyl) benzyl am, Polyethylene glycol ether of linear secondary alcohol - iodine complex Polyhexamethylene biguanidine Polymeric quaternary ammonium chloride, Polyoxyethylene (10 moles), nonyl phenol-formaldehyde resin, Polyoxyethylene (12 moles) methylene bis octophenol Polyoxyethylene (15) coconut amine, Polyoxyethylene (16 moles) hydroabietyl alcohol, Polyoxyethylene (18 moles) methylene bis (diamyl phenol), Polyoxyethylene (7-7.5 moles) isopropylidene diphenol, Polyoxyethylene amylphenol-formaldehyde resin (10 moles), Polyoxyethylene condensate with abietylamine, Polyoxyethylene dinonyl phenol, Polyoxyethylene dodecylphenol (6-12 moles), Polyoxyethylene octadecyl phenol, Polyoxyethylene p-tert-butyl phenol-formaldehyde resin, Polyvinyl pyrrolidone, Polyvinyl pyrrolidone - iodine complex, Polyvinylpyrrolidone-formaldehyde complex, Polyvinylpyrrolidone-orthophenylphenol complex, Poly[hydroxyethylene(dimethyliminio)ethylene(dimethyliminio)methylene dichloride], Potassium 2,2'-methylenebis(3,4,6-trichlorophenate), Potassium 2,4,6-trichlorophenate, Potassium 3,4-dichloroisothiazole-5-carboxylate, Potassium acetate, Potassium ammonium ethylenebis(dithiocarbamate), Potassium bisulfate, Potassium bromide, Potassium carbonate, Potassium chlorate, Potassium dimethyl dithio carbamate, Potassium dodecylbenzene sulphonate, Potassium glycolate, Potassium hydroxide, Potassium hypochlorite, Potassium iodide, Potassium laurate, Potassium laurate and myristate soaps, Potassium laurate, other related Potassium nitrate Potassium nitrite, Potassium para-tert-butylphenate, Potassium permanganate, Potassium peroxydisulfate, Potassium peroxymonosulfate, Potassium peroxymonosulfate sulfate, (2KHSO5.KHSO4.K2SO4), Potassium phosphate, dibasic Potassium pyrophosphate, Potassium silicate, Potassium toluene sulphonate, Potassium triiodide, Potassium xylenesulfonate, Probenazole, Propineb, Propionic acid, Propyl paraben Propyl paraben calcium salt, Propyl paraben sodium salt, Propylene glycol, Quinoline derivatives S-(2-hydroxypropyl) thiomethanesulfonate, Salicylanilide, Salicylic acid, Silica gel, Silver acetate, Silver carbonate, Silver chloride, Silver copper zeolite, Silver fluoride, Silver iodide, colloidal Silver nitrate, Silver orthophosphate (Ag3PO4), Silver oxide (Ag4O4), Silver salt of partially polymerized mannuronic acid, Silver sodium hydrogen zirconium phosphate (Ag0.18Na0.57H0.25Zr2(PO4)3), Silver thiocyanate, Silver thiuronium acrylate co-polymer, Silver zeolite, Silver zinc zeolite, Silver, ionic Soap, Sodium 1-naphthalenesulfonate, Sodium 2,2'-methylenebis(4-chlorophenate), Sodium 2-ethylhexanoate, Sodium 2-naphthalenesulfonate, Sodium acetate, Sodium acid phosphate, Sodium alkyl naphthalene sulphonate, Sodium alkyl* benzene sulfonate *(100% C9), Sodium alkylaryl sulphonate, Sodium benzene sulphonate, Sodium binoxalate, Sodium bisulfate, Sodium bromide, Sodium butyl naphthalene sulphonate, Sodium carbonate, Sodium chlorate, Sodium chloride, Sodium chlorite, Sodium cresolate, Sodium decyl benzene sulphonate, Sodium dibutyl naphthalene sulphonate, Sodium dichloro-s-triazinetrione, Sodium dichloro-s-triazinetrione dehydrate, Sodium dichlorophenate, Sodium dihydroxyethylglycine, Sodium diisobutyl naphthalene sulphonate, Sodium diisopropyl naphthalene sulphonate, Sodium dimethyl naphthalene sulphonate, Sodium dodecylbenzene sulphonate, Sodium dodecylbenzene sulfonate - iodine complex, Sodium formaldehyde sulfoxylate, Sodium glycolate, Sodium hydroxide, Sodium hydroxymethylamino acetate, Sodium hypobromite, Sodium hypochlorite, Sodium isopropyl naphthalene sulphonate, Sodium laurate, Sodium lauryl sulfate, Sodium metasilicate, Sodium metasilicate, anhydrous Sodium methyl naphthalene sulphonate, Sodium methyl nonyl naphthalene sulphonate, Sodium methylundecyl benzenesulfonate ,Sodium mono and dimethyl naphthalene sulphonate, Sodium N-alkyl-N-palmitoyl taurate-iodine complex, Sodium N-cyclohexyl-N-palmitoyl taurate - iodine complex, Sodium N-methyl-N-oleyl laurate, Sodium naphthalene sulphonate, Sodium nitrate, Sodium nitrite, Sodium nonanoyloxybenzene sulphonate, Sodium octylbenzene sulphonate, Sodium oxalate, Sodium p-chloro-m-cresolate, Sodium p-toluene sulfonyl chloramide, Sodium para-tert-amylphenate, Sodium para-tert-butylphenate, Sodium peroxide (Na2(O2)), Sodium phenate, Sodium phosphate, Sodium polyethoxyethyl dodecylsulfate, Sodium propionate, Sodium pyrithione, Sodium sesquicarbonate, Sodium silicate, Sodium toluene sulphonate, Sodium tridecylbenzene sulphonate, Sodium triisopropyl naphthalene sulphonate, Sodium triphenyl benzene sulphonate, Stearyl imidazoline, Sterilix, Streptomycin, Strontium dodecyl benzene sulphonate, Sulfamic acid, Sulfuric acid, Tall oil fatty acid soap of N-alkyl (C16-C18) propyl diamine TCMTB Temasept-R, other related Terbuthylazine, Terpineols, Tertbutylphenol oxyethylated adduct, Tetradecyl dimethyl benzyl ammonium chloride, Tetradecyl dimethyl benzyl ammonium chloride dehydrate, Tetraglycine hydroperiodide, Tetrahydro abietylamine acetate, Tetrakis (hydroxymethyl) phosphonium sulfate, Tetrasodium pyrophosphate, Toluene sulfonic acid, Trans-1,2-bis (n-propylsulfonyl) ethane, Tri-n-butyl tetradecyl phosphonium chloride, Tribromsalan, Tributyltin acetate, Tributyltin acrylate, Tributyltin benzoate, Tributyltin chloride, Tributyltin chloride complex of ethylene oxide condensate of abietylamine, Tributyltin chloride myristylamine salt Tributyltin fluoride, Tributyltin isopropyl succinate, Tributyltin linoleate, Tributyltin maleate, Tributyltin methacrylate, Tributyltin monopropylene glycol maleate, Tributyltin neodecanoate, Tributyltin oxide, Tributyltin resinate, Tributyltin sulfide, Trichloro melamine, Trichloro-s-triazinetrione, Trichlorophenol sodium salt, other related Triclosan, Tricopper dichloride dimethyldithiocarbamate, Tridecylpoly(ethyleneoxy)ethanol - iodine complex, Triethanoalmine nonyl phenyl poly oxyethylene sulfuric acid, Triethanolamine, Triethanolamine borate, Triethanolamine complex (see DPR chem code 1615), Triethanolamine dodecylbenzene sulphonate, Triethanolamine ester of fish oil acids, Triethanolamine laurate, Triethanolamine laurate-myristate, Triethanolamine lauryl sulfate, Triethanolamine myristate, Triethanolamine octylsulfate - iodine complex, Triethanolamine phosphate, Triethanolamine sulphonate, Triethanolamine sulfonate tridecylpolyoxyethyleneethanol - bromine complex, Triethanolamine thiocyanate, Triethylene glycol, Trioxymethylene, Tripotassium phosphate, Tris (hydroxymethyl) nitromethane, Trisodium phosphate, Turkey red oil, Turkey red oil sodium salt, Undecyl benzene sulfonic acid triethanolammonium salt, Urea, Vancide 30-R, Xylene ,Zinc 2-pyridinethiol-1-oxide, Zinc 8-quinolinolate, Zinc bacitracin, Zinc chloride, Zinc dehydroabietylammonium 2-ethylhexanoate, Zinc dodecyl benzene sulphonate, Zinc silicate, Zinc sulfate heptahydrate, Zinc sulfate, and anhydrous Zinc trichlorophenate Ziram.

In one embodiment, the at least one active ingredient is chosen from a group consisting of halogenated biocides. Exemplary halogenated biocides include, but are not limited to, 2,2-Dibromo-3-nitrilopropionamide (DBNPA), 2-Bromo-2-nitropropene-1,3-diol (BNPD), 3-iodo-2-propynylbutyl carbamate (IPBC), Chlorohexidine gluconate, chloroisocyanurates, chlorothalonil, halogenated hydantoins, and iodophors.

In another embodiment, the at least one active ingredient is chosen from a group consisting of inorganic biocides. Exemplary inorganic biocides include, but are not limited to, cuprous oxide and inorgano-silver.

In yet another embodiment, the at least one active ingredient is chosen from a group consisting of nitrogen-based biocides. Exemplary nitrogen-based biocides includes, but are not limited to, N-(3,4-dichlorophenyl)-N',N'-dimethylurea (diuron), methyene-bis-morpholine (MBM), quaternary ammonium compounds (quats), salicylamide, and triazines.

In still another embodiment, the at least one active ingredient is chosen from a group consisting of organometallics. Exemplary organometallics include, but are not limited to, 10,10'-ozybisphenoxerside (OBPA), bis(tributyltin) oxide (TBTO), tributyltin-chloride (TBTC), and triphenyltin chloride (TPTC).

In another embodiment, the at least one active ingredient is chosen from a group consisting of organometallic biocides. Exemplary organometallic biocides include, but are not limited to, disodium ethylenebis, dithiocarbemate, potassium dimethyldithiocarbamate, sodium dimethyldithiocarbamate, 1,2-benzisothiaxolin-3-one, 5-chloro-2-methyl-4-isothiazolin-3-one and 2-methyl-4-isothiazolin-3-in-one (CIT/MIT), 4,5 dichloro-2-n-octyl-4-isothiazolin-3-one (DCOIT), 2-n-octyl-4-isothiozolin-3-one (OIT),N-butyl-1,2-benzisothiazolin-3-one (BBIT), zinc-2-pyridinethiol-2-oxide (ZPT), methylenebis (tiocyanate) (MBT), 2-(thiocyanomethylthio)benzothiazole (TCMTB), tetrahydro-3,5-dimethyl-2H-1,3,5-thiadiazine-2-thione (thione), (5-chloro-2,4-dichlorophenoxyl) phenol (triclosan),o-Phenylphenol (OPP), glutaraldehyde, and peracetic acid.

In yet another embodiment, the at least one active ingredient is chosen from a group consisting of phenolic biocides. Exemplary phenolic biocides include, but are not limited to, (5-chloro-2,4-dichlorophenoxyl) phenol (triclosan), 3,4,4'-trichlorocarbanilide (triclocarban), o-Benzo-p-chlorophenol (OBCP), o-phenylphenol (OPP), pentachlorophenol (PCP), phenoxyethanol, and p-hydroxybenzoates (parabens).

In another embodiment, the at least one active ingredient is chosen from the group consisting of antimicrobial agents and preservatives. Exemplary antimicrobial agents and preservatives include, but are not limited to, in-can preservatives, film preservatives, wood preservatives, fibre preservatives, leather preservatives, rubber preservatives, polymerized materials preservatives, masonry preservatives, liquid cooling system preservatives, processing system preservatives, slimicides, metalworking-fluid preservatives, food preservatives, feedstock preservatives, phenoxyethanol, triclosan, 7-ethylbicyclooxazolidine, benzoic acid, bronopol (e.g., 2-bromo-2-nitropropane-13-diol), butylparaben, chlorite, chlorphenesin, diazolidinyl urea, dichlorobenzyl alcohol, dimethyl oxazolidine, DMDM hydantoin, ethylparaben, hexamidine diisethionate, imidiazolidinyl urea, imidiazolidinyl urea NF, iodopropynyl butylcarbamate, isobutylparaben, methylparaben, potassium sorbate NF FCC, propylparaben, quaternium-15, sodium benzoate NF FCC, sodium caprylate, sodium dehydroacetate, sodium dehydroacetate FCC, sodium hydroymethylglycinate, sodium hydroxymethylglycinate, sodium methylparaben, sodium propylparaben, sorbic acid NF FCC, anisic acid, benzethonium chloride, caprylic/capric glycerides, caprylyl glycol, di-alpha-tocopherol, ethylhexylglycerin, glyceryl caprate, methyl isothiazolinone, polymethoxy bicyclic oxazolidine, Tocopheryl acetate, alcohol, benzalkonium chloride, benzethonium chloride, camellia sinensis leaf extract, candida bombicola/glucose/ methyl rapeseedate, hydrogen peroxide, methylbenzethonium chloride phenol, pinus pinaster bark extract, Poloxamer 188, PVP-Iodine, Rosmarinus officinalis Leaf extract, Vitis vinifera seed extract, ammomium benzoate, ammonium propioante, 5-Bromo-5-nitro-1,3-dioxane, Chloroxylenol, Ethyl alcohol, Glutaral, Iodopropynyl butylcarabamate, Isothiazolinone, Parabens, Pircotone olamine, Selenium disulphine, Sorbic acid (mold), Zinc pyrithione, Benzalkonium chloride, Benzethonium chloride, Benzoic acid, Dehydroacetic acid, Dimethyl hydroxmethylpyrazole, Formaldehyde, Hexetidine, Methyldibromo glutaronitrile, Salicylic acid, Sodium hydroxymethylglycinate, Sodium iodate, Zinc oxide, Benzyl alcohol (mould), Boric acid (yeast), Chloroacetamide, Phenoxythanol, Orthophenylphenol, Benzalkonium chloride, Benzethonium chloride, 5-Bromo-5-nitro-1,3-dioxane, Bronopol, Diazolidinyl urea, Dimethyl hydroxmethylpyrazole, Dimethyl oxazolidine, DMDM hydantoin, Ethyl alcohol, 7-Ethyl bicycloxazolidine, Glutaral, Imidazolidinyl urea, Isothiazolinone, Methenammonium chloride, Methylbromo glutaronitrile, Polymethoxy bicylooxazolidine, Quaternium-15, Sodium hydroxymethylglycinate, Thimersal, Benzoic acid, Benzyl alcohol, Chlorhexidine, Hexetidine, Phenethyl alcohol, Polyaminopropyl biguanide, Polyquarternium-42, Salicylic acid, Sodium iodate, Triclocarban, Zinc phenolsulphonate, Chloroacetamide, Chlorobutanol, Dehydroacetic acid, Neem seed oil, Phenoxyethanol, Tee trea oil, Usnic acid, Ammonim Benzoate, Ammonium Propionate, Benziosthiazolinone, Benzoic Acid, Benzotriazole, Benzyl Alcohol, Benzylhemiformal, Benylparaben, 5-Bromo-5-Nitro-1,3-Dioxane, 2-Bromo-2-Notropropane-1,3-Diol, Butyl Benzoate, Butylparaben, Calcium Benzoate, Calcium Paraben, Calcium Propionate, Calcium Salicylate, Calcium Sorbate, Captan, Chloramine T, Chlorhexidine Diacetate, Chlorhexidine Digluconate, Chlorhexidine Dithydrochloride, Chloroacetamine, Chlorobutanol, p-Chloro-m-Cresol, Chlorophene, p-Chlorophenol, Chlorothymol, Chloroxylenol, Citrus Grandis (Grapefruit) Fruit Extract, Citrus Grandis (Grapefruit) Seed Extract, Copper Usnate, m-Cresol, o-Cresol, p-Cresol, DEDM Hydantoin, DEDM Hydantoin Dilaurate, Dehydroacetic Acid, Diazolidinyl Urea, Dibromopropamidine Diisethionate, Dimethyl Hydroxymethyl Pyrazole, Dimethylol Ethylene Thiourea, Dimethyl Oxazolidine, Dithiomethylbenzamide, DMDM Hydantoin, DMHF, Domiphen Bromide, Ethyl Ferulate, Ethylparaben, Ferulic Acid, Glutaral, Glycerol Formal, Glyoxal, Hexamidine, Hexamidine Diparaben, Hexamidine Paraben, 4-Hydroxybenzoic Acid, Hydroxymethyl Dioxazabicyclooctane, Imidazolidinyl Urea, Iodopropynyl Butylcarbamate, Isobutylparaben, Isodecylparaben, Isopropyl Cresols, Isopropylparaben, Isopropyl Sorbate, Magnesium Benzoate, Magnesium Propionate, Magnesium Salicylate, MDM Hydantoin, MEA-Benzoate, MEA o-Phenylphenate, MEA-Salicylate, Methylchloroisthiazolinone, Methyldibromo Glutaronitrile, Methylisothazolinone, Methylparaben, Mixed Cresols, Nisin, PEG-5 DEDM Hydantoin, PEG-15 DEDM Hydantoin, PEG-5 Hydantoin Oleate, PEG-15 DEDM Hydantoin Stearate, Phenethyl Alcohol, Phenol, Phenoxyethanol, Phenoxyethylparaben, Phenoxyisopropanol, Phenyl Benzoate, Phenyl Mercuric Acetate, Phenyl Mercuric Benzoate, Phenyl Mercuric Borate, Phenyl Mercuric Bromide, Phenyl Mercuric Chloride, Phenylparaben, Polyaminopropyl Biguanide, Polyaminopropyl Biguanide Stearate, Polymethoxy Bicyclic Oxazolidine, Polyquaternium-42, Potassium Benzoate, Potassium Ethylparaben, Potassium Methylparaben, Potassium Paraben, Potassium Phenoxide, Potassium o-Phenylphenate, Potassium Propionate, Potassium Propylparaben, Potassium Salicylate, Potassium Sorbate, Propionic Acid, Propyl Benzoate, Propylparaben, Quaternium-8, Quaternium-14, Quaternium-15, Silver Borosilicate, Silver Magnesium Aluminium Phosphate, Sodium Benzoate, Sodium Butylparaben, Sodium p-Chloro-m-Cresol, Sodium Dehydroacetate, Sodium Ethylparaben, Sodium Formate, Sodium Hydroxymethane Sulfonate, Sodium Hydroxymethylglycinate, Sodium Isobutylparaben, Sodium Methylparaben, Sodium Paraben, Sodium Phenolsulfonate, Sodium Phenoxide, Sodium o-Phenylphenate, Sodium Propionate, Sodium Propylparaben, Sodium Pyrithione, Sodium Salicylate, Sodium Sorbate, Sorbic Acid, TEA-Sorbate, Thimerosal, Triclocarban, Triclosan, Undecylenoyl PEG-5 Paraben, Zinc Pyrithione or combinations thereof, such as for example Benzyl Alcohol/mehtylchloroisothiazolinone/ methylisothiazolinone, Benzyl alcohol/PPG-2 methyl ether/bronopol/deceth-8/iodopropynyl/butylcarbamate, Chloroacetamide sodium benzoate, Dehydroacetic acid/benzyl alcohol, Diazolidinyl urea/iodopropynyl butylcarbamate, Diazolidinyl urea/methylparaben/ethylparaben/butylparaben/propylparaben/isobutylparabe- n/2-phenoxyethanol, DMDM hydantoin/iodopropynyl butylcarbamate, Glycerin/water/ ethoxdiglycol/caprylyl glycol/sodium polyacrylate, Glyceryl laurate/caprylyl/ phenylpropanol/dipropylene glycol, imidazole, Isopropylparaben/isobutylparaben/butylparaben, Methyl chloroisothiazolinone/methyl isothiazolinone, Methyldibromo glutaronitrile/ methylchloroisothiazolinone/methylisothiazolinone/phenoxye-thanol, Methyldibromo glutaronitrile/phenoxyethanol, Methylchloroisothiazolinone/methylisothiazolinone, Methylparaben/ethylparaben/butylparaben/propylparaben/butylenes glycol, Methylparaben/ethylparaben/butylparaben/propylparaben/isobutylparaben, Methylparaben/ethylparaben/butylparaben/propylparaben/isobutylparaben/2-phenoxyethanol/bronopol, Methylparaben/ethylparaben/butylparaben/propylparaben/1,3-butylene glycol isomer, Methylparaben/propylparaben, Methylparaben/propylparaben/benzyl alcohol, Methylparaben/propylparaben/bronopol/phenoxyethanol,

Methylparaben/propylparaben/bronopol/propylene glycol, Methylparaben/propylparaben/ethylparaben, Methylparaben/propylparaben/propylene glycol/diazolidinyl urea, oxazolidines, 2-phenylphenol, 2,4,4'-trichloro-2'-hydroxy diphenol ether, diiodomethyl-p-tolylsulfone, N-alkyl-N,N-dimethyl-N-benzylammonium chloride, zinc 2-mercaptopyridine-N-oxide, Phenoxyethanol/benzoic acid/dehydroacetic acid, Phenoxyethanol/benzyl alcohol/potassium sorbate/tocopherol, Phenoxylethanol/chlorphenesin/glycerin/methylparaben/benzoic acid, Phenoxyethanol/DMDM hydantoin/lodopropynyl butyl carbamate, Phenoxyethanol/DMDM hydantoin/methylparaben/propylparaben, Phenoxyethanol/isopropylparaben/isobutylparaben/butylparaben, Phenoxyethanol/methyldibromo glutaronitrile/idopropynyl butylcarbamate, Phenoxyethanol/methylparaben/butylparaben/ethylparaben/propylparaben, Phenoxyethanol/methylparaben/butylparaben/ethylparaben/propylparaben/isob-utyl-paraben, Phenoxyethanol/methylparaben/isobutylparaben/butylparaben, Phenoxythanol/triethylene glycol/dichlorobenzyl alcohol, Polyaminopropyl biguanide/parabens/phenoxyethanol, PPG-2 methyl ether/sodium benzoate/potassium sorbate/iodopropynyl butylcarbamate, Propylene glycol/benzyl alcohol/methylchloroisothiazolinone/methylisothiazolinone, Propylene glycol/diazolidinyl urea/iodopropynyl butylcarbamate, Propylene glycol/diazolidinyl urea/methylparaben/propylparaben, Propylene glycol/MDMD hydantoin/methylparaben, Propylene glycol/MDMD hydantoin/methylparaben/propylparaben, Propylene glycol/lichen extract, Propylene glycol/phenoxyethanol/chlorphenesin/methylparaben, and Sodium levulinate/phenylpropanol combinations.

In another embodiment, the active ingredient is an antibiotic. Exemplary antibiotics include, but are not limited to, Beta-lactam, Vancomycin, Macrolides, Tetracyclines, Quinolones, Fluoroquinolones, Nitrated compounds, Aminoglycosides, Phenicols, Lincosamids, Synergistins, Fosfomycin, Fusidic acid, oxazolidinones, Rifamycins, Polymixynes, Gramicidins, Tyrocydine, Glycopeptides, Sulfonamides and Trimethoprims In a further embodiment, the active ingredient comprises at least one antibiotic and at least one antimicrobial agent. In yet another embodiment, the active ingredient is a fungicide. In yet a further embodiment, the active ingredient is a mildewcide.

Methods of Adding Active Ingredients to Surface Treated Functional Particulate Carrier Materials

At least one active ingredient may be added to, and caused to bind to or otherwise interact with, at least one functional particulate carrier material that has been subjected to at least one surface treatment through various methods now known to the skilled artisan or hereafter discovered. In one embodiment, a powdered sample of at least one surface treated functional particulate carrier material is surface-saturated with at least one active ingredient and subjected to vacuum to drive off air from the pores of the particulate functional carrier material, thus allowing the at least one active ingredient to interact with the surface of the at least one surface treated functional particulate carrier material. In another embodiment, a powdered sample of at least one surface treated functional particulate carrier material is surface-saturated with at least one active ingredient and at least one fluid (e.g., deionized water), which is then shaken overnight at a temperature of about 50°C. The recovered samples may then be centrifuged to further separate the attached active ingredients from the unreacted powder, and the mixing and centrifugation steps may be repeated multiple times. In a further embodiment, a powdered sample of at least one surface treated functional particulate carrier material is surface-saturated with at least one active ingredient and subjected to vacuum to drive off air from the pores of the particulate functional carrier material, and the sample is then dried at about 105 °C overnight. In yet another embodiment, the at least one active ingredient is added to the at least one particulate functional carrier material that has been subjected to at least one surface treatment such that a series of covalent bonds links the at least one active ingredient, the at least one surface treating agent, and the at least one particulate functional carrier material. In yet a further embodiment, the at least one surface treated particulate functional carrier material absorbs the at least one active ingredient. In still another embodiment, the at least one active ingredient adsorbs onto the surface of the at least one surface treated particulate functional carrier material. In another embodiment, the at least one surface treated particulate functional carrier material interacts with the at least one active ingredient such that a timed and/or extended-release surface treated functional particulate carrier material is formed.

The at least one active ingredient may be added to the at least one particulate functional carrier material in any amount now known or hereafter discovered by the skilled artisan. In one embodiment, the at least one active ingredient is added in an amount that will achieve the results intended or desired by the presence of the at least one active ingredient. In another embodiment, the at least one active ingredient is added in an amount ranging from about 0.1% to about 2.0% by weight of the at least one particulate functional carrier material. In a further embodiment, the at least one active ingredient is added in an amount less than about 2.0% by weight. In yet another embodiment, the at least one active ingredient is added in an amount of about 0.5% by weight. In yet a further embodiment, the at least one active ingredient is added in an amount of about 1.0% by weight.

Microorganisms

Surface treated functional particulate carrier materials with at least one active ingredient may exhibit increased antimicrobial activity according to the present disclosure and, therefore, can be used for the inhibition of the growth and progeny of at least one microorganism. Microorganisms include, but are not limited to, bacteria (gram positive and gram negative bacteria), yeasts, fungi, mildew, viruses, and combinations thereof. Exemplary microorganisms include, but are not limited to, *Staphylococci, Micrococci, Escherichia, Pseudomonas, Bacilli, Salmonella, Shigella, Porphyromonas, Prevotella, Wolinella, Campylabacter, Propionibacterium, Streptococci. Cprumebacterium, Treponema, Fusobacterium, Bifidobacterium, Lactobacillus, Actinomyces, Candida, Malazessia,* and *Aspergillus.* The skilled artisan readily understands that the embodiments disclosed herein may effectively inhibit the growth of any microorganism, depending upon the at least one active ingredient added to the surface treated functional particulate carrier material.

Uses for Surface Treated Carrier Materials with Active Ingredient

The surface treated functional particulate carrier materials with at least one active ingredient may be used in any application now known to the skilled artisan or hereafter discovered, in which enhanced performance of the application is desired though increased retention of the at least one active ingredient. In one embodiment, the surface treated functional particulate carrier materials with at least one active ingredient are used in formulations or applications, including, but not limited to, animal feed, cosmetic formulations, paints, inks, home care products, animal care products, building materials, paper products, fabric products (e.g., textiles), products for personal and work hygiene, contact lenses, chromatography materials, medical equipment, protective topicals, pharmaceutical and especially dermatological formulations, lacquers, coatings, polymers, and plastics.

Exemplary formulations and applications include, but are not limited to, adhesives, sealants, antimicrobial cleansers, soaps, disinfectants, anti-fouling and antimicrobial paints for inside and outside use, anti-foulant marine coatings, animal husbandry products, antimicrobial wallpapers, antimicrobial dressings and plasters, prostheses and bone cement with antimicrobial activity, dental fillings, dental prostheses, formulations against gastrointestinal infections, active coal, antimicrobial cat litter, air conditioning (filters and ducts), air inflated construction (air halls), agricultural and mulch films, all purpose adhesives, appliances and equipment, appliance adhesives and sealants, aprons, artificial leather, artificial plants, artificial wood, and plastic lumber, Astroturf, automobile parts, automotive and truck upholstery, awnings, bags, bandages, barrier fabrics, bathroom accessories, bathtubs, bathtub cement, bedding, beverage dispensers, bibs, boats, boat covers, book covers, bottles, brush bristles, brush handles, brooms, building components (walls, wallboard, floors, concrete, siding, roofing, shingles, hardware, carpet cleaner, ceilings and commercial and industrial applications), cable sheathing, caps (hats), cardboard, carpet and carpet underlay, caster wheels, cat litter, clinical thermometers, coats, compact discs, convertible tops, cookware, coolers, cooling towers, cooling water, counter and table tops, conveyor belts, countertops, credit cards, crates (food and non-food), cups, currency, curtains, cushion pads, cutting boards, decking, dishes, dish cloths, dishwasher components, diving equipment or snorkels, drainage sewer pipe, draperies, dry-film paints, exercise equipment, equipment for slaughterhouses or creameries or diaries, equipment for gyms, saunas or massages, fan blades, fiberfill, filters, fittings, fences, floor coverings, floor and carpet baking, flooring, foam (cushion, mattress), food preparation appliances, food and beverage processing equipment, food and drink containers, storage and bags, food handling equipment, food packaging, food and meat crates, food trays and covers, food wrap, footwear (including boots, sports equipment, and tools), fruit and vegetable brushes, fruit crates, furniture, garbage bags, garbage cans, garment bags, gaskets, general purpose containers, gloves, gowns (medical and consumers), grease traps, rigid greenhouses, greenhouse films, grout and joint compound, heating, ventilation and air conditioning, hospital surface and medical instrument disinfection, hoses, ice-making equipment and trays, in-can paints, incontinence care products, indoor and outdoor furniture, industrial equipment, inflatable bed, insulation for wire and cable, insulators, intimate apparel, jacket liners, janitorial equipment, kitchen and bathroom hardware, kitchen sinks and fixtures, kitchen towels, laminate and tile adhesives, laying batteries, life vests, liners, mats, mattress cover pads and filing, mattress adhesives, medical and dental apparel, metal working fluids, mineral slurries, mobile homes, mobile toilets, mops, money, natural and synthetic fibers and fabrics, non-woven fabrics, oilfield, outerwear, packaging, pallets, paper products (wipes, tissues, wall coverings, towels, book covers, mulch), pillow covers, pipes, pipe sealant and insulating materials, plaster, plastics, plastic films, plates and utensils, playground equipment, plumbing supplies and fixtures (including toilet bowl seats), plumbing adhesives and sealants, pool liners, process vessels, protective covers,recreational water, resins, refrigerator components, roofing sheets, membranes, shingles and flashing, ropes, rugs, sales counter, sails, sanitary pipes, sealers, sealing compounds for bathrooms, kitchens or glass, sheets and blankets, shoes, shoe insoles, shower curtains, shower tubs, siding for housing, silage wrap, silos, sinks, siphons, skylights, sleeping bags, sleepwear, socks and hosiery, sponges, sprinkler, sportswear and sports equipment, storage containers, stucco, sun roof, sun shades, synthetic latex polymers, napkins, tanks, tape, tarps, telephone boxes or public phones, tents and other outdoor equipment, ticking (mattress pillow), tiles, tile grout, toothbrush handle and bristles, toilet paper and handkerchiefs, toilet blocks and cleaners, towels, toothbrush tumbler, toys, trim for outerwear and garments, trunk liners, tubing, umbrellas, undergarments, uniforms, upholstery, vacuum cleaner bags, wall and floor covering, wallpaper, waste bags, water tanks, waste containers, water treatment, water and ice handling equipment and filters, wet suits, wipes, wire and cable, wood, wood filled plastics.

In several application areas, enhanced biocidal or antimicrobial activity may be useful in several stages of the processing. In one embodiment, plastics and/or polymers comprising surface treated functional particulate carrier materials with at least one active ingredient and exhibiting enhanced antimicrobial or biocidal capabilities according to the present disclosure can be stored in the form of Masterbatches for a long period of time, without risking the contamination of the Masterbatch with microorganisms. The skilled artisan recognizes that the Masterbatch can be processed in the same way as known Masterbatches, or in processing methods hereafter discovered. The treated Masterbatches may be useful in, for example, building and construction, household, items and furnishing, electrical and electronics parts, apparel, textiles and fabrics, coatings and laminates, transportation and recreation, adhesives, sealants and grouts, food contact items and water contact items (such as plastic bottles and bottle caps), films, coextrusion films, and exterior and interior automotive parts.

In another embodiment, suitable plastics and polymers from which the articles may be fabricated comprising in part the surface treated functional particulate carrier material with at least one active ingredient may include synthetic, natural, and semisynthetic organic polymers. Examples of polymers include, but are not limited to: aliphatic and aromatic polyesters, including polyethylene terephthalate, polybutylene terephthalate, polyethylene isophthalate, polyhexamethylene terephthalate, polylactic acid, polyglycolic acid, and liquid crystalline polymers for high performance resins and fibers; polyester block copolymers; aliphatic and aromatic polyamides including nylon 6, nylon 66, nylon 610, nylon 11, nylon 12, nylon 1212, poly-p-phenylene terephthalamide, poly-m-phenylene isophthalamide; copolymerised polyamides; polyolefins including polyethylene, polypropylene, and copolymers thereof; vinyl polymers, including polystyrene, polyacrylonitrile, polyvinylalcohol, polyvinyl acetate, polyvinylchloride, polyvinylidene chloride, ABS resins and acrylic resins; copolymers of ethylene and vinyl acetate; fluorocarbon polymers, including polytetrafluoroethylene. polyvinylidene fluoride and polyvinyl fluoride; polyurethanes; segmented polyurethane elastomers, spandex or elastane elastomers; polyethers, including polyacetals; polyketones, polyetherether ketone (PEEK), polyether ketoneketone (PEKK); polyether and polyester block polymers; polysulfides; polysulfones: polysiloxanes such as polydimethyl siloxane; polycarbonates; thermosetting synthetic polymers such as phenol-formaldehyde copolymer, polyurethane, polyesterurethane, polyetherurethane, polyetherurethaneurea, polyesterurethaneurea; natural polymers such as cellulosics, cotton and wool; and regenerated or semi-synthetic polymers such as rayon, cuprammonium rayon, acetate rayon, triacetate rayon, reconstituted silk and polysaccharides. Copolymers, terpolymers, and blends of species listed are also contemplated.

In a further embodiment, waterborne paints and lacquers may comprise surface treated functional particulate carrier materials with at least one active ingredient. In yet another embodiment, solvent-based paints and lacquers may comprise the surface treated functional particulate carrier materials with at least one active ingredient.

Enhanced Antimicrobial Activity

The surface treated functional particulate carrier materials of the present disclosure may retain larger quantities of the at least one active ingredient, which may in turn enhance the antimicrobial and/or biocidal activity of the materials. In one embodiment, improved antimicrobial activity is measured by the retention factors (Rf) of the functional particulate carrier materials by testing with methods known in the art, such as thin layer chromatography, which may measure the concentration of the released active ingredient, by which the difference of the added and measured Rf may then be taken. In another embodiment, improved antimicrobial activity is measured by the retention factors of the functional particulate carrier materials by adding about 0.75 g of the at least one active ingredient dropwise to about 2.5 g of the functional particulate carrier material, mixing for about 8 hours, charging with about 1000 mL of water, and stirring continuously for about 90 minutes until the elution curve plateaus. The suspension is then filtered and the water is analyzed for released biocide by any appropriate detection technique, such as 2nd derivative UV-Vis spectroscopy or Gas Chromatography Mass Spectrometry. Such an embodiment may yield Rf values from about 5% to about 250% for siliceous materials, and may yield Rf values of about 0% for diatomaceous earth not treated according to the present disclosure.

In the disclosure disclosed herein, a larger Rf generally shows that the surface treated functional particulate carrier materials retain larger quantities of the at least one active ingredient compared to functional particulate carrier materials that were not surface treated. The higher retention rate reveals increased antimicrobial activity in these surface treated functional particulate carrier materials.

## EXAMPLES

### Example 1

Five control samples of powdered, non-surface treated functional particulate carrier materials were tested against two powdered functional particulate carrier material samples that had been subjected to surface treatment by silanization. Then, the two non-control samples were then surface-saturated with the active ingredient biocide, Neem oil. After various drying and separation techniques were performed, as described below, the retention factors of the seven samples were measured.

The functional particulate carrier material of Sample 1 was CelTiX™, a diatomaceous earth product available from World Minerals, Inc. Sample 1 was not subjected to vacuum pressure and was not surface treated before Neem oil was added by the process described below. Sample 1 had a retention factor of 0.

The functional particulate carrier material of Sample 2 was CelTiX™, as in Sample 1. Sample 2 was subjected to vacuum pressure but was not surface treated before Neem oil was added by the process described below. Sample 2 had a retention factor of 0.

The functional particulate carrier material of Sample 3 was Superfloss™, a super-fine diatomaceous earth product sold by Celite Corp., a subsidiary of World Minerals, Inc. Sample 3 was not subjected to vacuum pressure and was not surface treated before Neem oil was added by the process described below. Sample 3 had a retention factor of 0.

The functional particulate carrier material of Sample 4 was Micro-Cel E, a synthetic calcium silicate hydrate ($CaSiO_3$) available from Advanced Minerals Corp., a subsidiary of World Minerals Inc. Sample 4 was not subjected to vacuum pressure and was not surface treated before Neem oil was added by the process described below. Sample 4 had a retention factor of 78.

The functional particulate carrier material of Sample 5 was Harborlite 635, a very fine grade of perlite available from Harborlite Corp., a subsidiary of World Minerals Inc. Sample 5 was not subjected to vacuum pressure and was not surface treated before Neem oil was added by the process described below. Sample 5 had a retention factor of 154.

The functional particulate carrier material of Sample 6 was CelTiX™. Sample 6 was subjected to surface treatment by silanization as described below with a 1% solution of vinyltriethoxysilane, available from GE Silicone under the designation "Silquest® A-151 Silane," before Neem oil was added by the process described below. Sample 6 had a retention factor of 330.

In the retention experiments, a powdered sample was first surface-saturated with Neem oil by mixing 2 gram of a sample with 10 grams of Neem oil, vacuum was also used to drive off air from the pores so that the oil can get into them. After the treatment, 200 ml of DI water was than added into the flask, and shaken well to mix the contents, the sample was then placed in a shaker set at 50°C for overnight. The recovered samples were then centrifuged to further separate the attached oil from the carrier powders. The mixing and centrifugation were repeated for three times. The samples were then dried at 105°C for overnight, followed by ignition at 800°C, to burn off any retained Neem oil. After these treatments, the original untreated DE samples failed to retain the oil, most of the oil were separated from the powders. The surface-treated samples, however, showed much higher retentions.

The functional particulate carrier material of Sample 7 was CelTiX™. Sample 7 was subjected to vacuum pressure and was subjected to surface treatment by silanization as described below with a 1% solution of 3-aminopropyl triethoxysilane, available from GE Silicones under the designation "Silquest A-1100 Silane," before Neem oil was added by the process described below. Sample 7 had a retention factor of 317.

For the surface treatment by silanization, an appropriate amount of silane was added dropwise to the dry inorganic carrier. For these examples, approximately 10 g of silane was charged to 1000 g of carrier and the mixture was blended in a V-shaped Rota-Vee Tumble powder blender for at least 60 minutes.

For the addition of Neem oil, in a flask, two grams of each sample were surface-saturated with 10 grams of Neem oil. Samples 2, 6, and 7 were subjected to vacuum pressure of 20 mm Hg to drive off air from the pores of the particulate functional carrier to allow the oil to better penetrate the sample. Then, 200 milliliters of deionized water was added into the flask and shaken well to mix the contents. The samples were placed in a shaker set at 50 °C overnight. The recovered samples were centrifuged using a Beckman J6-MI Centrifuge at 3000 RPM for 20 minutes to further separate the attached oil from the powder. The mixing and centrifugation steps were repeated three times. The samples were dried at 105 °C overnight to drive off moisture, followed by ignition at 800 °C to burn off the retained Neem oil. The percentage weight loss after the ignition was taken as the weight percentage of retained Neem oil, as follows:

$$R_f = (W_2 - W_1)*100/W_1$$

where $W_1$ was the sample weight after drying at 105°C, $W_2$ was the sample weight after ignition.

The five control samples of different functional particulate carriers were tested against the two silanized samples. After the treatments, the un-silanized samples failed to retain the oil and most of the oil separated from the powders, regardless of the presence or absence of vacuum pressure. The surface treated samples, however, showed much higher retention. Table 1 shows these results.

Table 1

|  | Vacuum | Surface Treatment | Retention Factor (%) |
|---|---|---|---|
| **Sample 1** | NO | NO | 0 |
| **Sample 2** | YES | NO | 0 |
| **Sample 3** | NO | NO | 0 |
| **Sample 4** | NO | NO | 78 |
| **Sample 5** | NO | NO | 154 |
| **Sample 6** | YES | YES | 330 |
| **Sample 7** | YES | YES | 317 |

Surprisingly and unexpectedly, surface treatment in accordance with the present inventions resulted in greatly improved neem oil retention, as shown in Table 1.

**Example 2**

In this example, DE materials that were subjected to a surface treatment in accordance with the present inventions were used, as well as non-surface treated DE for comparison. As noted in Table 2, 1.5 g samples of DE were mixed with 2 mL Skane™ M-8 (mildewcide available from Rohm and Haas Company), and then placed into a vacuum oven set at 50°C for 10 minutes. The samples were then vigorously washed with water to wash off unretained biocide, by mixing the samples with 50 mL of deionized water and shaking at 50°C and 200RPM for two hours, followed by filtering. The filtered DE cake was then subjected to the same washing process twice more. After the third washing, the DE cake was dried and ignited at 700°C to burn off organics. The ignition loss is compared with a blank DE sample and taken as the percent retention of biocide in Table 2.

"A-1101" is a gamma-aminopropyltriethoxysilane sold by GE Silicones under the name Silquest® A-1101, and is a technical grade of Silquest® A-1100.

"A-151" is a vinyltriethoxysilane sold by GE Silicones under the name Silquest® A-151 Silane.

"PMDS" is a polydimethylsiloxane sold by Wacker Chemical Corporation under the name AK1000.

Table 2

| DE | Surface Treatment | Mildewcide | % Mildewcide Retained |
|---|---|---|---|
| Superfloss™ | None | None | 0.00 |
| | None | Skane™ M-8 | 14.89 |
| | 0.6% A-1101 | Skane™ M-8 | 22.26 |
| | 0.6% A-151 | Skane™ M-8 | 26.15 |
| | 1% PDMS | Skane™ M-8 | 29.49 |
| CelTiX™ | None | None | 0.00 |
| | None | Skane™ M-8 | 1.78 |
| | 1% A-1101 | Skane™ M-8 | 14.54 |
| | 1% A-151 | Skane™ M-8 | 36.74 |
| | 1% PMDS | Skane™ M-8 | 17.97 |

Surprisingly and unexpectedly, surface treatment in accordance with the present inventions resulted in greatly improved mildewcide retention, as shown in Table 2.

**Claims**

1. A surface treated functional particulate carrier material, comprising:

   at least one functional particulate carrier material subjected to at least one surface treatment; and,
   at least one active ingredient,
   wherein the at least one functional particulate carrier material is chosen from the group consisting of synthetic calcium silicate hydrate, salts thereof, and diatomaceous earth and wherein the at least one surface treatment is a chemical surface modification with at least one surface treating agent chosen from the group consisting of at least one silane wherein the at least one silane is trialkoxysilane, and wherein the at least one surface treated functional particulate carrier material absorbs the at least one active ingredient or the at least one active ingredient adsorbs onto the surface of the at least one surface treated functional particulate carrier material.

2. The material according to claim 1, wherein the at least one functional particulate carrier material is diatomaceous earth.

3. The material according to claim 1, wherein the at least one silane is vinyltriethoxysilane.

4. The material according to claim 1, wherein the at least one silane is 3-aminopropyl triethoxysilane.

5. The material according to any preceding claim, wherein at least one active ingredient is chosen from the group consisting of biocides, antibiotics, fungicides, mildewcides, insecticides, preservatives, and antimicrobial agents.

6. The material according to claim 5, wherein the biocide is chosen from the group consisting of Neem oil, isothiazolinones, silver oxides, silver salts, copper oxides, copper salts, zinc oxide, zinc salts, and iodopopargyl butyl carbamate.

7. The material according to claim 1, wherein the at least one functional particulate carrier material is diatomaceous earth, the at least one silane is vinyltriethoxysilane, and the at least one active ingredient is Neem oil.

8. The material according to claim 1, wherein the at least one functional particulate carrier material is diatomaceous earth, the at least one silane is 3-aminopropyl triethoxysilane, and the at least one active ingredient is Neem oil.

9. A cosmetic formulation, a paint, an ink, a home care product, an animal care product, a personal hygiene product, a work hygiene product, a contact lens, a chromatography material, medical equipment, a protective topical, a pharmaceutical formulation, a dermatological formulation, a lacquer, a coating, a polymer, or a plastic comprising the material according to any preceding claim.

10. A surface treated functional particulate carrier material according to claim 1, wherein the at least one active ingredient is chosen from at least one biocide, and wherein the material has a retention factor of at least 100, wherein the retention factor of the functional particulate carrier material is measured by adding about 0.75g of the at least one active ingredient dropwise to about 2.5g of the functional particulate carrier material, mixing for about 8 hours, charging with about 1000mL of water, and stirring continuously for about 90 minutes until the elution curve plateaus, filtering the suspension and analysing the water for released biocide by Gas Chromatography Mass Spectrometry.

11. A method of making a surface treated functional particulate carrier material according to claim 1, wherein the at least one functional particulate carrier material is surface treated to chemically modify its surface prior to exposure to the at least one active ingredient.

**Patentansprüche**

1. Oberflächenbehandeltes Funktionsteilchenträgermaterial, umfassend:

mindestens ein Funktionsteilchenträgermaterial, das mindestens einer Oberflächenbehandlung unterzogen wurde; und, mindestens einen Wirkstoff, wobei das Funktionsteilchenträgermaterial aus synthetischem Calciumsilicathydrat, dessen Salzen und/oder Diatomeenerde besteht und wobei es sich bei der mindestens einen Oberflächenbehandlung um eine chemische Oberflächenmodifizierung mit mindestens einem Oberflächenbehandlungsmittel handelt, gewählt aus der Gruppe bestehend aus mindestens einem Silan handelt, wobei es sich bei dem mindestens einen Silan um Trialkoxysilan handelt und wobei das mindestens eine oberflächenbehandelte Funktionsteilchenträgermaterial den mindestens einen Wirkstoff absorbiert oder der mindestens eine Wirkstoff in die Oberfläche des mindestens einen oberflächenbehandelten Funktionsteilchenträgermaterials adsorbiert.

2. Material gemäß Anspruch 1, wobei es sich bei dem mindestens einen Funktionsteilchenträgermaterial um Diatomeenerde handelt.

3. Material gemäß Anspruch 1, wobei es sich bei dem mindestens einen Silan um Vinyltriethoxysilan handelt.

4. Material gemäß Anspruch 1, wobei es sich bei dem mindestens einen Silan um 3-Aminopropyltriethoxysilan handelt.

5. Material gemäß einem der vorhergehenden Ansprüche, wobei mindestens ein Wirkstoff aus Bioziden, Antibiotika, Fungiziden, Schimmelverhütungsmitteln, Insektiziden, Konservierungsmitteln und/oder antimikrobiellen Mitteln besteht.

6. Material gemäß Anspruch 5, wobei das Biozid aus Margosaöl, Isothiazolinonen, Silberoxiden, Silbersalzen, Kupferoxiden, Kupfersalzen, Zinkoxid, Zinksalzen und/oder Iodopopargylbutylcarbamat besteht.

7. Material gemäß Anspruch 1, wobei es sich bei mindestens einem Funktionsteilchenträgermaterial um Diatomeenerde, bei dem mindestens einen Silan um Vinyltriethoxysilan und bei dem mindestens einen Wirkstoff um Margosaöl handelt.

8. Material gemäß Anspruch 1, wobei es sich bei mindestens einem Funktionsteilchenträgermaterial um Diatomeenerde, bei mindestens einem Silan um 3-Aminopropyltriethoxysilan und bei mindestens einem Wirkstoff um Margosaöl handelt.

9. Kosmetische Formulierung, Farbe, Tinte, Haushaltspflegeprodukt, Tierpflegeprodukt, Körperpflegeprodukt, Arbeitshygieneprodukt, Kontaktlinse, chromatographisches Material, medizinische Ausrüstung, schützendes topisches Mittel, pharmazeutische Formulierung, dermatologische Formulierung, Lack, Beschichtung, Polymer oder Kunststoff, der/die/das Material gemäß einem der vorhergehenden Ansprüche umfasst.

**10.** Oberflächenbehandeltes Funktionsteilchenträgermaterial gemäß Anspruch 1, wobei der mindestens eine Wirkstoff aus mindestens einem Biozid besteht,
und wobei das Material einen Retentionsfaktor von mindestens 100 aufweist, wobei der Retentionsfaktor des Funktionsteilchenträgermaterials gemessen wird, indem etwa 0,75 g von mindestens einem Wirkstoff tropfenweise zu etwa 2,5 g des Funktionsteilchenträgermaterials gegeben werden, diese für ungefähr 8 Stunden gemischt, ungefähr 1000 ml Wasser hinzugegeben und für ungefähr 90 Minuten kontinuierlich gerührt werden, bis die Elutionskurve abflacht, die Suspension filtrieren und das Wasser durch Gaschromatographie mit Massenspektrometrie-Kopplung auf freigesetztes Biozid analysieren.

**11.** Verfahren zur Herstellung eines oberflächenbehandelten Funktionsteilchenträgermaterials gemäß Anspruch 1, wobei das mindestens eine Funktionsteilchenträgermaterial oberflächenbehandelt wird, um dessen Oberfläche chemisch zu modifizieren, bevor es dem mindestens einen Wirkstoff ausgesetzt wird.

**Revendications**

**1.** Un matériau véhiculaire particulaire fonctionnel traité en surface, comprenant :

au moins un matériau véhiculaire particulaire fonctionnel soumis à au moins un traitement de surface ; et
au moins un ingrédient actif,
dans lequel au moins un matériau véhiculaire particulaire fonctionnel est choisi dans le groupe constitué d'hydrate de silicate de calcium synthétique, ses sels, et la terre de diatomées et dans lequel au moins un traitement de surface est une modification chimique de surface avec au moins un agent de traitement de surface choisi dans le groupe constitué d'au moins un silane, dans lequel au moins un silane est le trialkoxysilane, et dans lequel au moins un matériau véhiculaire particulaire fonctionnel traité en surface absorbe au moins un ingrédient actif ou au moins un ingrédient actif s'absorbe sur la surface du au moins un matériau véhiculaire particulaire fonctionnel traité en surface.

**2.** Le matériau selon la revendication 1, dans lequel au moins un matériau véhiculaire particulaire fonctionnel est de la terre de diatomées.

**3.** Le matériau selon la revendication 1, dans lequel au moins un silane est du vinyltriéthoxysilane.

**4.** Le matériau selon la revendication 1, dans lequel au moins un silane est le 3-aminopropyl triéthoxysilane.

**5.** Le matériau selon l'une quelconque des revendications précédentes, dans lequel au moins un ingrédient actif est choisi dans le groupe constitué par les biocides, les antibiotiques, les fongicides, les agents anticryptogamiques, les insecticides, les conservateurs et les agents antimicrobiens.

**6.** Le matériau selon la revendication 5, dans lequel le biocide est choisi dans le groupe constitué par l'huile de Neem, les isothiazolinones, les oxydes d'argent, les sels d'argent, les oxydes de cuivre, les sels de cuivre, l'oxyde de zinc, les sels de zinc, et le carbamate d'iodopopargylbutyle.

**7.** Le matériau selon la revendication 1, dans lequel au moins un matériau véhiculaire particulaire fonctionnel est de la terre de diatomées, au moins un silane est du vinyltriéthoxysilane et au moins un ingrédient actif est de l'huile de Neem.

**8.** Le matériau selon la revendication 1, dans lequel au moins un matériau véhiculaire particulaire fonctionnel est de la terre de diatomées, au moins un silane est du 3-aminopropyl triéthoxysilane, et au moins un ingrédient actif est de l'huile de Neem.

**9.** Une formulation cosmétique, une peinture, une encre, un produit de soins domestiques, un produit de soin des animaux, un produit d'hygiène personnelle, un produit d'hygiène du travail, une lentille cornéenne, un matériau chromatographique, un équipement médical, un topique protecteur, une formulation pharmaceutique, une formulation dermatologique, une laque, un revêtement, un polymère, ou un plastique comprenant le matériau selon l'une quelconque des revendications précédentes.

**10.** Un matériau véhiculaire particulaire fonctionnel traité en surface selon la revendication 1, dans lequel au moins un ingrédient actif est choisi parmi au moins un biocide, et dans lequel le matériau a un facteur de rétention d'au moins 100, dans lequel le facteur de rétention du matériau véhiculaire particulaire fonctionnel est mesuré en ajoutant environ 0,75 g d'au moins un ingrédient actif en goutte à environ 2,5 g du matériau véhiculaire particulaire fonctionnel, en mélangeant pendant environ 8 heures, en chargeant avec environ 1 000 mL d'eau, et en remuant continuellement pendant environ 90 minutes jusqu'à obtenir un plateau dans la courbe d'élution, en filtrant la suspension et en analysant l'eau pour le biocide libéré par chromatographie en phase gazeuse couplée à la spectrométrie de masse

11. Un procédé de fabrication d'un matériau véhiculaire particulaire fonctionnel traité en surface selon la revendication 1, dans lequel au moins un matériau véhiculaire particulaire fonctionnel est traité en surface pour modifier chimiquement sa surface avant l'exposition à au moins un ingrédient actif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6905698 B1
- US 4505889 A
- US 4552591 A
- US 5648086 A
- US 7018643 B
- US 20060035097 A
- US 20060180552 A
- US 20060246149 A1
- US 4656057 A